# EUROPEAN PATENT SPECIFICATION

(11) **EP 2 201 113 B1**
(45) Date of publication and mention of the grant of the patent: **16.09.2015**
(21) Application number: 08839874.8
(22) Date of filing: 17.10.2008
(51) Int. Cl.: G01N 33/68

(54) **METHOD OF IDENTIFYING AGENTS WHICH MODULATE THE ACTIVITY OF CALCIUM-ACTIVATED CHLORIDE CHANNEL**
VERFAHREN ZUR IDENTIFIKATION VON MITTELN ZUR MODULIERUNG DER AKTIVITÄT VON CALCIUM-AKTIVIERTEN CHLORIDKANÄLEN
PROCÉDÉ D'IDENTIFICATION DES AGENTS MODULANT L'ACTIVITÉ D'UN CANAL CHLORURÉ ACTIVÉ PAR LE CALCIUM

(30) Priority: 18.10.2007 US 980850 P
(43) Date of publication of application: 30.06.2010
(73) Proprietor: SNU R&DB Foundation, Seoul 151-742 (KR)
(72) Inventor: OH, Uhtaek, Seongnam-si Gyeonggi-do 463-763 (KR); CHO, Hawon, Seoul 138-751 (KR); YANG, Young Duk, Seoul 151-704 (KR); TAK, Min Ho, Seoul 135-814 (KR); JANG, Yongwoo, Busan 614-819 (KR); KOO, Jae Yeon, Seoul 140-748 (KR); CHO, Si Young, Seoul 136-761 (KR); JEONG, Yeon Su, Yongin-si Gyeonggi-do 448-749 (KR)
(74) Representative: Grünecker Patent- und Rechtsanwälte PartG mbB
(86) International application number: PCT/KR2008/006146
(87) International publication number: WO 2009/051439

(56) References cited:
- US-B1- 6 377 057
- SCHROEDER BJOERN CHRISTIAN ET AL: "Expression cloning of TMEM16A as a calcium-activated chloride channel subunit", CELL, vol. 134, no. 6, September 2008 (2008-09), pages 1019-1029, XP002636497, ISSN: 0092-8674
- CAPUTO ANTONELLA ET AL: "TMEM16A, a membrane protein associated with calcium-dependent chloride channel activity", SCIENCE (WASHINGTON D C), vol. 322, no. 5901, 4 September 2008 (2008-09-04), pages 590-594, XP002636498, ISSN: 0036-8075
- HARTZELL C. ET AL.: 'Calcium-Activated Chloride Channels.' ANNUAL REVIEWS PHYSIOLOGY. vol. 67, March 2005, pages 719 - 758, XP008132052
- HECHT J. ET AL.: 'Detection of novel skeletogenesis target genes by comprehensive analysis of a Runx2(-/-) mouse model' GENE EXPRESSION PATTERNS vol. 7, 06 June 2006, pages 102 - 112, XP005763805
- VISEL A. ET AL.: 'GenePaint.org: an atlas of gene expression patterns in the mouse embryo' NUCLEIC ACIDS RESEARCH vol. 32, 01 January 2004, pages D552 - 556, XP008132051
- YOUNG DUK YANG ET AL.: 'TMEM16A confers receptor-activated calcium-dependent chloride conductance.' NATURE vol. 455, 24 August 2008, pages 1210 - 1215, XP008132054

### SEQUENCE LISTING

<110> AMOREPACIFIC CORPORATION
<120> METHOD OF IDENTIFYING AGENTS WHICH MODULATE THE ACTIVITY OF CALCIUM-ACTIVATED CHLORIDE CHANNEL
<130> PCA71096
<150> US 60/980,850
   <151> 2007-10-18
<160> 17
<170> KopatentIn 1.71
<210> 1
   <211> 960
   <212> PRT
   <213> Mus musculus
<400> 1
   Met Arg Val Pro Glu Lys Tyr Ser Thr Leu Pro Ala Glu Asp Arg Ser
   1 5 10 15
   Val His Ile Val Asn Ile Cys Ala Ile Glu Asp Leu Gly Tyr Leu Pro
   20 25 30
   Ser Glu Gly Thr Leu Leu Asn Ser Leu Ser Val Asp Pro Asp Ala Glu
   35 40 45
   Cys Lys Tyr Gly Leu Tyr Phe Arg Asp Gly Lys Arg Lys Val Asp Tyr
   50 55 60
   Ile Leu Val Tyr His His Lys Arg Ala Ser Gly Ser Arg Thr Leu Ala
   65 70 75 80
   Arg Arg Gly Leu Gln Asn Asp Met Val Leu Gly Thr Arg Ser Val Arg
   85 90 95
   Gln Asp Gln Pro Leu Pro Gly Lys Gly Ser Pro Val Asp Ala Gly Ser
   100 105 110
   Pro Glu Val Pro Met Asp Tyr His Glu Asp Asp Lys Arg Phe Arg Arg
   115 120 125
   Glu Glu Tyr Glu Gly Asn Leu Leu Glu Ala Gly Leu Glu Leu Glu Asn
   130 135 140
   Asp Glu Asp Thr Lys Ile His Gly Val Gly Phe Val Lys Ile His Ala
   145 150 155 160
   Pro Trp His Val Leu Cys Arg Glu Ala Glu Phe Leu Lys Leu Lys Met
   165 170 175
   Pro Thr Lys Lys Val Tyr His Ile Ser Glu Thr Arg Gly Leu Leu Lys
   180 185 190
   Thr Ile Asn Ser Val Leu Gln Lys Ile Thr Asp Pro Ile Gln Pro Lys
   195 200 205
   Val Ala Glu His Arg Pro Gln Thr Thr Lys Arg Leu Ser Tyr Pro Phe
   210 215 220
   Ser Arg Glu Lys Gln His Leu Phe Asp Leu Thr Asp Arg Asp Ser Phe
   225 230 235 240
   Phe Asp Ser Lys Thr Arg Ser Thr Ile Val Tyr Glu Ile Leu Lys Arg
   245 250 255
   Thr Thr Cys Thr Lys Ala Lys Tyr Ser Met Gly Ile Thr Ser Leu Leu
   260 265 270
   Ala Asn Gly Val Tyr Ser Ala Ala Tyr Pro Leu His Asp Gly Asp Tyr
   275 280 285
   Glu Gly Asp Asn Val Glu Phe Asn Asp Arg Lys Leu Leu Tyr Glu Glu
   290 295 300
   Trp Ala Ser Tyr Gly Val Phe Tyr Lys Tyr Gln Pro Ile Asp Leu Val
   305 310 315 320
   Arg Lys Tyr Phe Gly Glu Lys Val Gly Leu Tyr Phe Ala Trp Leu Gly
   325 330 335
   Ala Tyr Thr Gln Met Leu Ile Pro Ala Ser Ile Val Gly Val Ile Val
   340 345 350
   Phe Leu Tyr Gly Cys Ala Thr Val Asp Glu Asn Ile Pro Ser Met Glu
   355 360 365
   Met Cys Asp Gln Arg Tyr Asn Ile Thr Met Cys Pro Leu Cys Asp Lys
   370 375 380
   Thr Cys Ser Tyr Trp Lys Met Ser Ser Ala Cys Ala Thr Ala Arg Ala
   385 390 395 400
   Ser His Leu Phe Asp Asn Pro Ala Thr Val Phe Phe Ser Val Phe Met
   405 410 415
   Ala Leu Trp Ala Ala Thr Phe Met Glu His Trp Lys Arg Lys Gln Met
   420 425 430
   Arg Leu Asn Tyr Arg Trp Asp Leu Thr Gly Phe Glu Glu Glu Glu Glu
   435 440 445
   Ala Val Lys Asp His Pro Arg Ala Glu Tyr Glu Ala Arg Val Leu Glu
   450 455 460
   Lys Ser Leu Arg Lys Glu Ser Arg Asn Lys Glu Thr Asp Lys Val Lys
   465 470 475 480
   Leu Thr Trp Arg Asp Arg Phe Pro Ala Tyr Phe Thr Asn Leu Val Ser
   485 490 495
   Ile Ile Phe Met Ile Ala Val Thr Phe Ala Ile Val Leu Gly Val Ile
   500 505 510
   Ile Tyr Arg Ile Ser Thr Ala Ala Ala Leu Ala Met Asn Ser Pro Pro
   515 520 525
   Ser Val Arg Ser Asn Ile Arg Val Thr Val Thr Ala Thr Ala Val Ile
   530 535 540
   Ile Asn Leu Val Val Ile Ile Leu Leu Asp Glu Val Tyr Gly Cys Ile
   545 550 555 560
   Ala Arg Trp Leu Thr Lys Ile Glu Val Pro Lys Thr Glu Lys Ser Phe
   565 570 575
   Glu Glu Arg Leu Thr Phe Lys Ala Phe Leu Leu Lys Phe Val Asn Ser
   580 585 590
   Tyr Thr Pro Ile Phe Tyr Val Ala Phe Phe Lys Gly Arg Phe Val Gly
   595 600 605
   Arg Pro Gly Asp Tyr Val Tyr Ile Phe Arg Ser Phe Arg Met Glu Glu
   610 615 620
   Cys Ala Pro Gly Gly Cys Leu Met Glu Leu Cys Ile Gln Leu Ser Ile
   625 630 635 640
   Ile Met Leu Gly Lys Gln Leu Ile Gln Asn Asn Leu Phe Glu Ile Gly
   645 650 655
   Ile Pro Lys Met Lys Lys Phe Ile Arg Tyr Leu Lys Leu Arg Arg Gln
   660 665 670
   Ser Pro Ser Asp Arg Glu Glu Tyr Val Lys Arg Lys Gln Arg Tyr Glu
   675 680 685
   Val Asp Phe Asn Leu Glu Pro Phe Ala Gly Leu Thr Pro Glu Tyr Met
   690 695 700
   Glu Met Ile Ile Gln Phe Gly Phe Val Thr Leu Phe Val Ala Ser Phe
   705 710 715 720
   Pro Leu Ala Pro Leu Phe Ala Leu Leu Asn Asn Ile Ile Glu Ile Arg
   725 730 735
   Leu Asp Ala Lys Lys Phe Val Thr Glu Leu Arg Arg Pro Val Ala Ile
   740 745 750
   Arg Ala Lys Asp Ile Gly Ile Trp Tyr Asn Ile Leu Arg Gly Val Gly
   755 760 765
   Lys Leu Ala Val Ile Ile Asn Ala Phe Val Ile Ser Phe Thr Ser Asp
   770 775 780
   Phe Ile Pro Arg Leu Val Tyr Leu Tyr Met Tyr Ser Gln Asn Gly Thr
   785 790 795 800
   Met His Gly Phe Val Asn His Thr Leu Ser Ser Phe Asn Val Ser Asp
   805 810 815
   Phe Gln Asn Gly Thr Ala Pro Asn Asp Pro Leu Asp Leu Gly Tyr Glu
   820 825 830
   Val Gln Ile Cys Arg Tyr Lys Asp Tyr Arg Glu Pro Pro Trp Ser Glu
   835 840 845
   His Lys Tyr Asp Ile Ser Lys Asp Phe Trp Ala Val Leu Ala Ala Arg
   850 855 860
   Leu Ala Phe Val Ile Val Phe Gln Asn Leu Val Met Phe Met Ser Asp
   865 870 875 880
   Phe Val Asp Trp Val Ile Pro Asp Ile Pro Lys Asp Ile Ser Gln Gln
   885 890 895
   Ile His Lys Glu Lys Val Leu Met Val Glu Leu Phe Met Arg Glu Glu
   900 905 910
   Gln Gly Lys Gln Gln Leu Leu Asp Thr Trp Met Glu Lys Glu Lys Pro
   915 920 925
   Arg Asp Val Pro Cys Asn Asn His Ser Pro Thr Thr His Pro Glu Ala
   930 935 940
   Gly Asp Gly Ser Pro Val Pro Ser Tyr Glu Tyr His Gly Asp Ala Leu
   945 950 955 960
<210> 2
   <211> 2883
   <212> DNA
   <213> Mus musculus
<400> 2
   atgagggtcc ccgagaagta ctcgacgctc ccggcggagg accgcagcgt ccacatcgtg 60
   aacatctgcg ccatcgagga cctgggctac ctgccgtccg agggcacgtt gctgaactct 120
   ctgtccgtgg accccgacgc cgaatgcaag tatggactat acttcaggga tggcaaacgg 180
   aaggtggact acatcttggt ataccatcac aagagagcct cagggagcag gactctggcc 240
   aggaggggac tacaaaatga catggtcctg gggacccgca gcgtcaggca ggaccagccc 300
   cttcccggga aggggagccc tgtggatgca ggctcaccgg aagtccccat ggattaccat 360
   gaagatgaca aacgcttcag acgggaggaa tatgagggca acctgttgga ggcaggcctg 420
   gagttggaga atgacgagga taccaaaatc catggtgtcg ggtttgtgaa gatccatgcg 480
   ccctggcatg tgctctgtag ggaagctgag tttttgaaac taaagatgcc cacaaagaag 540
   gtgtaccaca tcagtgagac gcgaggcctc ctgaaaacca tcaactcggt tctgcagaag 600
   atcacagacc ccatccagcc caaggtggct gagcacaggc cacagaccac aaagaggctc 660
   tcctatccct tctcccggga gaagcaacac ctattcgacc tgactgacag ggactctttt 720
   ttcgacagca aaacccggag cacaatagtc tatgaaatcc tgaagagaac aacgtgcacc 780
   aaggccaagt acagcatggg tatcaccagc ctcctggcca atggcgtata ctcagctgca 840
   taccctctgc acgatgggga ctatgagggt gacaacgttg agttcaacga caggaaactc 900
   ctgtatgagg aatgggcaag ttacggagtc ttctacaaat accagcccat tgacctggtc 960
   aggaaatact ttggtgagaa ggttggcctg tactttgcct ggcttggagc ctacacccag 1020
   atgctcatcc ctgcctcgat cgtgggtgtc attgtctttc tctatggatg tgccactgtg 1080
   gacgaaaaca tccccagtat ggagatgtgt gaccagagat acaacatcac catgtgtcct 1140
   ctgtgtgaca agacctgcag ctactggaag atgagctcag cctgtgccac agcccgtgcc 1200
   agtcaccttt ttgataaccc tgccaccgtc ttcttctctg tgtttatggc cctctgggct 1260
   gccactttca tggagcactg gaaacggaag cagatgaggc tcaactaccg atgggacctc 1320
   acaggcttcg aggaggagga ggaagctgtc aaggatcatc ccagagcaga gtatgaagcc 1380
   agagtcttag agaagtcact gagaaaagaa tccagaaaca aagagaccga caaggtgaag 1440
   ctgacctgga gggaccgatt cccagcctat ttcaccaatc ttgtctccat catcttcatg 1500
   atcgcagtga catttgcaat cgtcctcgga gttatcatct atagaatctc cacagctgca 1560
   gccttggcca tgaactcccc cccgtctgtg cggtccaaca tccgggttac agtcacggcc 1620
   accgctgtta tcatcaacct cgtggtcatc attctgctgg atgaagttta cggctgcatt 1680
   gccaggtggc tcaccaagat tgaggtccca aagacagaga agagctttga ggagaggcta 1740
   accttcaagg ccttcctgct caagtttgtg aactcttaca ctcccatctt ctatgtcgcc 1800
   ttcttcaaag gccggtttgt tggtcggccc ggtgactacg tgtacatctt ccgctctttc 1860
   cggatggagg agtgtgcccc gggcggctgc ctcatggagc tctgtatcca gctgagcatc 1920
   attatgctgg gcaagcagct aatccagaac aatctcttcg agattggcat cccgaagatg 1980
   aaaaagttca tccgctacct gaagctgcgc agacagagcc cctcagaccg tgaagagtac 2040
   gtgaagcgga agcagcgcta tgaggtggac ttcaacctcg aacctttcgc cggcctcacg 2100
   cccgagtaca tggaaatgat cattcagttc ggctttgtca ccctgtttgt tgcgtccttc 2160
   cctctggctc cactcttcgc cctgctaaac aacatcattg agatccgcct ggatgccaaa 2220
   aagtttgtca ccgagctacg gaggccagta gccatcagag ccaaagacat cggcatctgg 2280
   tataacatcc tcagaggtgt tgggaagctg gctgtcatca ttaatgcctt tgtgatctcc 2340
   ttcacgtctg acttcatccc tcgcctggtg tacctctaca tgtacagtca gaatgggacc 2400
   atgcacggct tcgtcaatca cacgctctct tccttcaatg tcagcgactt ccagaatggc 2460
   acagcaccca atgacccact ggacctgggc tatgaggttc agatctgcag gtataaagat 2520
   taccgggaac ccccatggtc agaacacaag tatgacatct ccaaagactt ctgggctgtc 2580
   ctggccgccc gactggcatt tgtcattgtc ttccagaacc tggtgatgtt catgagtgac 2640
   tttgtggact gggtgatccc tgatatcccc aaagacatca gccagcagat ccacaaagag 2700
   aaggttctca tggtggagct gtttatgcgt gaggagcagg gcaagcagca gctactggac 2760
   acatggatgg agaaggagaa gccaagggat gtgccttgta acaaccacag ccccacaacc 2820
   cacccagagg caggcgacgg cagcccagtc cccagctacg agtaccatgg ggacgcgctg 2880
   tag 2883
<210> 3
   <211> 959
   <212> PRT
   <213> Homo sapiens
<400> 3
   Met Arg Val Asn Glu Lys Tyr Ser Thr Leu Pro Ala Glu Asp Arg Ser
   1 5 10 15
   Val His Ile Ile Asn Ile Cys Ala Ile Glu Asp Ile Gly Tyr Leu Pro
   20 25 30
   Ser Glu Gly Thr Leu Leu Asn Ser Leu Ser Val Asp Pro Asp Ala Glu
   35 40 45
   Cys Lys Tyr Gly Leu Tyr Phe Arg Asp Gly Arg Arg Lys Val Asp Tyr
   50 55 60
   Ile Leu Val Tyr His His Lys Arg Pro Ser Gly Asn Arg Thr Leu Val
   65 70 75 80
   Arg Arg Val Gln His Ser Asp Thr Pro Ser Gly Ala Arg Ser Val Lys
   85 90 95
   Gln Asp His Pro Leu Pro Gly Lys Gly Ala Ser Leu Asp Ala Gly Ser
   100 105 110
   Gly Glu Pro Pro Met Asp Tyr His Glu Asp Asp Lys Arg Phe Arg Arg
   115 120 125
   Glu Glu Tyr Glu Gly Asn Leu Leu Glu Ala Gly Leu Glu Leu Glu Arg
   130 135 140
   Asp Glu Asp Thr Lys Ile His Gly Val Gly Phe Val Lys Ile His Ala
   145 150 155 160
   Pro Trp Asn Val Leu Cys Arg Glu Ala Glu Phe Leu Lys Leu Lys Met
   165 170 175
   Pro Thr Lys Lys Met Tyr His Ile Asn Glu Thr Arg Gly Leu Leu Lys
   180 185 190
   Lys Ile Asn Ser Val Leu Gln Lys Ile Thr Asp Pro Ile Gln Pro Lys
   195 200 205
   Val Ala Glu His Arg Pro Gln Thr Met Lys Arg Leu Ser Tyr Pro Phe
   210 215 220
   Ser Arg Glu Lys Gln His Leu Phe Asp Leu Ser Asp Lys Asp Ser Phe
   225 230 235 240
   Phe Asp Ser Lys Thr Arg Ser Thr Ile Val Tyr Glu Ile Leu Lys Arg
   245 250 255
   Thr Thr Cys Thr Lys Ala Lys Tyr Ser Met Gly Ile Thr Ser Leu Leu
   260 265 270
   Ala Asn Gly Val Tyr Ala Ala Ala Tyr Pro Leu His Asp Gly Asp Tyr
   275 280 285
   Asn Gly Glu Asn Val Glu Phe Asn Asp Arg Lys Leu Leu Tyr Glu Glu
   290 295 300
   Trp Ala Arg Tyr Gly Val Phe Tyr Lys Tyr Gln Pro Ile Asp Leu Val
   305 310 315 320
   Arg Lys Tyr Phe Gly Glu Lys Ile Gly Leu Tyr Phe Ala Trp Leu Gly
   325 330 335
   Val Tyr Thr Gln Met Leu Ile Pro Ala Ser Ile Val Gly Ile Ile Val
   340 345 350
   Phe Leu Tyr Gly Cys Ala Thr Met Asp Glu Asn Ile Pro Ser Met Glu
   355 360 365
   Met Cys Asp Gln Arg His Asn Ile Thr Met Cys Pro Leu Cys Asp Lys
   370 375 380
   Thr Cys Ser Tyr Trp Lys Met Ser Ser Ala Cys Ala Thr Ala Arg Ala
   385 390 395 400
   Ser His Leu Phe Asp Asn Pro Ala Thr Val Phe Phe Ser Val Phe Met
   405 410 415
   Ala Leu Trp Ala Ala Thr Phe Met Glu His Trp Lys Arg Lys Gln Met
   420 425 430
   Arg Leu Asn Tyr Arg Trp Asp Leu Thr Gly Phe Glu Glu Glu Glu Glu
   435 440 445
   Ala Val Lys His Pro Arg Ala Glu Tyr Glu Ala Arg Val Leu Glu Lys
   450 455 460
   Ser Leu Lys Lys Glu Ser Arg Asn Lys Glu Thr Asp Lys Val Lys Leu
   465 470 475 480
   Thr Trp Arg Asp Arg Phe Pro Ala Tyr Leu Thr Asn Leu Val Ser Ile
   485 490 495
   Ile Phe Met Ile Ala Val Thr Phe Ala Ile Val Leu Gly Val Ile Ile
   500 505 510
   Tyr Arg Ile Ser Met Ala Ala Ala Leu Ala Met Asn Ser Ser Pro Ser
   515 520 525
   Val Arg Ser Asn Ile Arg Val Thr Val Thr Ala Thr Ala Val Ile Ile
   530 535 540
   Asn Leu Val Val Ile Ile Leu Leu Asp Glu Val Tyr Gly Cys Ile Ala
   545 550 555 560
   Arg Trp Leu Thr Lys Ile Glu Val Pro Lys Thr Glu Lys Ser Phe Glu
   565 570 575
   Glu Arg Leu Ile Phe Lys Ala Phe Leu Leu Lys Phe Val Asn Ser Tyr
   580 585 590
   Thr Pro Ile Phe Tyr Val Ala Phe Phe Lys Gly Arg Phe Val Gly Arg
   595 600 605
   Pro Gly Asp Tyr Val Tyr Ile Phe Arg Ser Phe Arg Met Glu Glu Cys
   610 615 620
   Ala Pro Gly Gly Cys Leu Met Glu Leu Cys Ile Gln Leu Ser Ile Ile
   625 630 635 640
   Met Leu Gly Lys Gln Leu Ile Gln Asn Asn Leu Phe Glu Ile Gly Ile
   645 650 655
   Pro Lys Met Lys Lys Leu Ile Arg Tyr Leu Lys Leu Lys Gln Gln Ser
   660 665 670
   Pro Pro Asp His Glu Glu Cys Val Lys Arg Lys Gln Arg Tyr Glu Val
   675 680 685
   Asp Tyr Asn Leu Glu Pro Phe Ala Gly Leu Thr Pro Glu Tyr Met Glu
   690 695 700
   Met Ile Ile Gln Phe Gly Phe Val Thr Leu Phe Val Ala Ser Phe Pro
   705 710 715 720
   Leu Ala Pro Leu Phe Ala Leu Leu Asn Asn Ile Ile Glu Ile Arg Leu
   725 730 735
   Asp Ala Lys Lys Phe Val Thr Glu Leu Arg Arg Pro Val Ala Val Arg
   740 745 750
   Ala Lys Asp Ile Gly Ile Trp Tyr Asn Ile Leu Arg Gly Ile Gly Lys
   755 760 765
   Leu Ala Val Ile Ile Asn Ala Phe Val Ile Ser Phe Thr Ser Asp Phe
   770 775 780
   Ile Pro Arg Leu Val Tyr Leu Tyr Met Tyr Ser Lys Asn Gly Thr Met
   785 790 795 800
   His Gly Phe Val Asn His Thr Leu Ser Ser Phe Asn Val Ser Asp Phe
   805 810 815
   Gln Asn Gly Thr Ala Pro Asn Asp Pro Leu Asp Leu Gly Tyr Glu Val
   820 825 830
   Gln Ile Cys Arg Tyr Lys Asp Tyr Arg Glu Pro Pro Trp Ser Glu Asn
   835 840 845
   Lys Tyr Asp Ile Ser Lys Asp Phe Trp Ala Val Leu Ala Ala Arg Leu
   850 855 860
   Ala Phe Val Ile Val Phe Gln Asn Leu Val Met Phe Met Ser Asp Phe
   865 870 875 880
   Val Asp Trp Val Ile Pro Asp Ile Pro Lys Asp Ile Ser Gln Gln Ile
   885 890 895
   His Lys Glu Lys Val Leu Met Val Glu Leu Phe Met Arg Glu Glu Gln
   900 905 910
   Asp Lys Gln Gln Leu Leu Glu Thr Trp Met Glu Lys Glu Arg Gln Lys
   915 920 925
   Asp Glu Pro Pro Cys Asn His His Asn Thr Lys Ala Cys Pro Asp Ser
   930 935 940
   Leu Gly Ser Pro Ala Pro Ser His Ala Tyr His Gly Gly Val Leu
   945 950 955
<210> 4
   <211> 4498
   <212> DNA
   <213> Homo sapiens
<400> 4
   agaggccgcc ggggccgtgg atggggaggg cgcgccgccc ggcggtccca gcgcacaggc    60
   ggccacgatg agggtcaacg agaagtactc gacgctcccg gccgaggacc gcagcgtcca   120
   catcatcaac atctgcgcca tcgaggacat cggctacctg ccgtccgagg gcacgctgct   180
   gaactcctta tctgtggacc ctgatgccga gtgcaagtat ggcctgtact tcagggacgg   240
   ccggcgcaag gtggactaca tcctggtgta ccatcacaag aggccctcgg gcaaccggac   300
   cctggtcagg agggtgcagc acagcgacac cccctctggg gctcgcagcg tcaagcagga   360
   ccaccccctg ccgggcaagg gggcgtcgct ggatgcaggc tcgggggagc ccccgatgga   420
   ctaccacgag gatgacaagc gcttccgcag ggaggagtac gagggcaacc tcctggaggc   480
   gggcctggag ctggagcggg acgaggacac taaaatccac ggagtcgggt ttgtgaaaat   540
   ccatgccccc tggaacgtgc tgtgcagaga ggccgagttt ctgaaactga agatgccgac   600
   gaagaagatg taccacatta atgagacccg tggcctcctg aaaaaaatca actctgtgct   660
   ccagaaaatc acagatccca tccagcccaa agtggctgag cacaggcccc agaccatgaa   720
   gagactctcc tatcccttct cccgggagaa gcagcatcta tttgacttgt ctgataagga 780
   ttcctttttc gacagcaaaa cccggagcac gattgtctat gagatcttga agagaacgac 840
   gtgtacaaag gccaagtaca gcatgggcat cacgagcctg ctggccaatg gtgtgtacgc 900
   ggctgcatac ccactgcacg atggagacta caacggtgaa aacgtcgagt tcaacgacag 960
   aaaactcctg tacgaagagt gggcacgcta tggagttttc tataagtacc agcccatcga 1020
   cctggtcagg aagtattttg gggagaagat cggcctgtac ttcgcctggc tgggcgtgta 1080
   cacccagatg ctcatccctg cctccatcgt gggaatcatt gtcttcctgt acggatgcgc 1140
   caccatggat gaaaacatcc ccagcatgga gatgtgtgac cagagacaca atatcaccat 1200
   gtgcccgctt tgcgacaaga cctgcagcta ctggaagatg agctcagcct gcgccacggc 1260
   ccgcgccagc cacctcttcg acaaccccgc cacggtcttc ttctctgtct tcatggccct 1320
   ctgggctgcc accttcatgg agcactggaa gcggaaacag atgcgactca actaccgctg 1380
   ggacctcacg ggctttgaag aggaagagga ggctgtcaag gatcatccta gagctgaata 1440
   cgaagccaga gtcttggaga agtctctgaa gaaagagtcc agaaacaaag agactgacaa 1500
   agtgaagctg acatggagag atcggttccc agcctacctc actaacttgg tctccatcat 1560
   cttcatgatt gcagtgacgt ttgccatcgt cctcggcgtc atcatctaca gaatctccat 1620
   ggccgccgcc ttggccatga actcctcccc ctccgtgcgg tccaacatcc gggtcacagt 1680
   cacagccacc gcagtcatca tcaacctagt ggtcatcatc ctcctggacg aggtgtatgg 1740
   ctgcatagcc cgatggctca ccaagatcga ggtcccaaag acggagaaaa gctttgagga 1800
   gaggctgatc ttcaaggctt tcctgctgaa gtttgtgaat tcctacaccc ccatctttta 1860
   cgtggcgttc ttcaaaggcc ggtttgttgg acgcccgggc gactacgtgt acattttccg 1920
   ttccttccga atggaagagt gtgcgccagg gggctgcctg atggagctat gcatccagct 1980
   cagcatcatc atgctgggga aacagctgat ccagaacaac ctgttcgaga tcggcatccc 2040
   gaagatgaag aagctcatcc gctacctgaa gctgaagcag cagagccccc ctgaccacga 2100
   ggagtgtgtg aagaggaaac agcggtacga ggtggattac aacctggagc ccttcgcggg 2160
   cctcacccca gagtacatgg aaatgatcat ccagtttggc ttcgtcaccc tgtttgtcgc 2220
   ctccttcccc ctggccccac tgtttgcgct gctgaacaac atcatcgaga tccgcctgga 2280
   cgccaaaaag tttgtcactg agctccgaag gccggtagct gtcagagcca aagacatcgg 2340
   aatctggtac aatatcctca gaggcattgg gaagcttgct gtcatcatca atgccttcgt 2400
   gatctccttc acgtctgact tcatcccgcg cctggtgtac ctctacatgt acagtaagaa 2460
   cgggaccatg cacggcttcg tcaaccacac cctctcctcc ttcaacgtca gtgacttcca 2520
   gaacggcacg gcccccaatg accccctgga cctgggctac gaggtgcaga tctgcaggta 2580
   taaagactac cgagagccgc cgtggtcgga aaacaagtac gacatctcca aggacttctg 2640
   ggccgtcctg gcagcccggc tggcgtttgt catcgtcttc cagaacctgg tcatgttcat 2700
   gagcgacttt gtggactggg tcatcccgga catccccaag gacatcagcc agcagatcca 2760
   caaggagaag gtgctcatgg tggagctgtt catgcgggag gagcaagaca agcagcagct 2820
   gctggaaacc tggatggaga aggagcggca gaaggacgag ccgccgtgca accaccacaa 2880
   caccaaagcc tgcccagaca gcctcggcag cccagccccc agccatgcct accacggggg 2940
   cgtcctgtag ctatgccagc ggggctgggc aggccagccg ggcatcctga ccgatgggca 3000
   ccctctccca gggcaggcgg cttcccgctc ccaccagggc ccggtgggtc ctgggttttc 3060
   tgcaaacatg gaggaccact ttctgatagg acattttcct ttcttctttc tgttttcttt 3120
   cccttgtttt tgcacaaagc cattatgcag ggaatatttt ttaatctgta gtattcaaga 3180
   tgaatcaaaa tgatggctgg taatacggca ataaggtagc aaaggcaggt gctttgcaga 3240
   aagaatgctt ggaaacttga gtctccctag aggtgaaaag tgagcagagg cccctagaaa 3300
   ccctcctctg aatcctccta attccttaag atagatgcaa aatggtaagc cgaggcatcg 3360
   cgcaaaagct ggtgcgatgc ttcagggaaa atggaaaacc cacgcaagaa taatgattga 3420
   ttccggttcc aaaaggtgtc acctacctgt ttcagaaaag ttagactttc catcgccttt 3480
   tccttccatc agttgagtgg ctgagagaga agtgcctcat ccctgagcca cacagggggc 3540
   gtgggagcat cccagttatc cctggaaagc tagaagggga cagaggtgtc cctgattaag 3600
   caggaaacag cacccttggc gtccccagca ggctccccac tgtcagccac acacctgccc 3660
   ccatcacacc aagccgacct cagagttgtt catcttcctt atgggacaaa accggttgac 3720
   cagaaaatgg gcagagagag atgacctcgg aagcatttcc acagatggtg tcagggtttc 3780
   aagaagtctt agggcttcca ggggtcccct ggaagcttta gaatatttat gggttttttt 3840
   ttcaaatatc aattatatgg tagattgagg attttttttc tgtagctcaa aggtggaggg 3900
   agtttattag ttaaccaaat atcgttgaga ggaatttaaa atactgttac taccaaagat 3960
   ttttattaat aaaggcttat attttggtaa cacttctcta tatttttact cacaggaatg 4020
   tcactgttgg acaattattt taaaagtgta taaaaccaag tctcataaat gatatgagtg 4080
   atctaaattt gcagcaatga tactaaacaa ctctctgaaa tttctcaagc accaagagaa 4140
   acatcatttt agcaaaggcc aggaggaaaa atagaaataa atttgtcttg aagatctcat 4200
   tgatgtgatg ttacattccc tttaatctgc caactgtggt caaagttcat aggtgtcgta 4260
   catttccatt atttgctaaa atcatgcaat ctgatgcttc tcttttctct tgtacagtaa 4320
   gtagtttgaa gtgggttttg tatataaata ctgtattaaa aattaggcaa ttaccaaaaa 4380
   tccttttatg gaaaccattt ttttaaaaag tgaatgtaca caaatccaca gaggactgtg 4440
   gctggacatt catctaaata aatttgaata tacgaaaaaa aaaaaaaaaa aaaaaaaa 4498
<210> 5
   <211> 1083
   <212> PRT
   <213> Rattus sp.
<400> : 5
   Met Gln Trp Gly Ala Gly Thr Gly Arg Gly Glu Gly Ala Ala Ala Gly
   1 5 10 15
   Asp Pro Val Thr Pro Gly Ala Glu Ser Trp Gly Asp Pro Arg Thr Lys
   20 25 30
   Ala Pro Gly Ala Glu Gln Arg Gly Ala Gly Gly Trp Ala Gln Pro Ser
   35 40 45
   Ser Arg Leu Cys Arg Ser Leu Cys Trp Thr Gln Gly Val Arg Ser Glu
   50 55 60
   His Ile Met Gln Asp Thr Gln Asp Ser Asp Ile Ser Leu Glu Gly Leu
   65 70 75 80
   Thr Arg Glu Gly Ala Pro Ala Asp Arg Glu Cys Gln Arg Gly Pro Glu
   85 90 95
   Thr Ile Val His Glu Ala Gln Asp Ala Gly Thr Pro Asn Ser Asp Ala
   100 105 110
   Thr Gly Ala Val Asp Gly Glu Arg Glu Ala Thr Met Arg Val Pro Glu
   115 120 125
   Lys Tyr Ser Thr Leu Pro Ala Glu Asp Arg Ser Val His Ile Val Asn
   130 135 140
   Ile Cys Ala Ile Glu Asp Leu Gly Tyr Leu Pro Ser Glu Gly Thr Leu
   145 150 155 160
   Leu Asn Ser Leu Ser Val Asp Pro Asp Ala Glu Cys Lys Tyr Gly Leu
   165 170 175
   Tyr Phe Arg Asp Gly Lys Arg Lys Val Asp Tyr Ile Leu Val Tyr His
   180 185 190
   His Lys Arg Ala Ser Gly Ser Arg Thr Leu Ala Arg Arg Gly Leu Gln
   195 200 205
   Asn Asp Met Val Leu Gly Thr Arg Ser Val Arg Gln Asp Gln Pro Leu
   210 215 220
   Pro Gly Lys Gly Ser Pro Val Asp Val Gly Ser Pro Glu Ala Pro Met
   225 230 235 240
   Asp Tyr His Glu Asp Asp Lys Arg Phe Arg Arg Glu Glu Tyr Glu Gly
   245 250 255
   Asn Leu Leu Glu Ala Gly Leu Glu Leu Glu Arg Asp Glu Asp Thr Lys
   260 265 270
   Ile His Gly Val Gly Phe Val Lys Ile His Ala Pro Trp His Val Leu
   275 280 285
   Cys Arg Glu Ala Glu Phe Leu Lys Leu Lys Met Pro Thr Lys Lys Val
   290 295 300
   Tyr His Ile Ser Glu Thr Arg Gly Leu Leu Lys Thr Ile Asn Ser Val
   305 310 315 320
   Leu Gln Lys Ile Thr Asp Pro Ile Gln Pro Lys Val Ala Glu His Arg
   325 330 335
   Pro Gln Thr Thr Lys Arg Leu Ser Tyr Pro Phe Ser Arg Glu Lys Gln
   340 345 350
   His Leu Phe Asp Leu Thr Asp Arg Asp Ser Phe Phe Asp Ser Lys Thr
   355 360 365
   Arg Ser Thr Ile Val Tyr Glu Ile Leu Lys Arg Thr Thr Cys Thr Lys
   370 375 380
   Ala Lys Tyr Ser Met Gly Ile Thr Ser Leu Leu Ala Asn Gly Val Tyr
   385 390 395 400
   Ser Ala Ala Tyr Pro Leu His Asp Gly Asp Tyr Glu Gly Asp Asn Val
   405 410 415
   Glu Phe Asn Asp Arg Lys Leu Leu Tyr Glu Glu Trp Ala Ser Tyr Gly
   420 425 430
   Val Phe Tyr Lys Tyr Gln Pro Ile Asp Leu Val Arg Lys Tyr Phe Gly
   435 440 445
   Glu Lys Val Gly Leu Tyr Phe Ala Trp Leu Gly Ala Tyr Thr Gln Met
   450 455 460
   Leu Ile Pro Ala Ser Ile Val Gly Val Ile Val Phe Leu Tyr Gly Cys
   465 470 475 480
   Ala Thr Val Asp Glu Asn Ile Pro Ser Met Glu Met Cys Asp Gln Arg
   485 490 495
   His Asn Ile Thr Met Cys Pro Leu Cys Asp Lys Thr Cys Ser Tyr Trp
   500 505 510
   Lys Met Ser Ser Ala Cys Ala Thr Ala Arg Ala Ser His Leu Phe Asp
   515 520 525
   Asn Pro Ala Thr Val Phe Phe Ser Val Phe Met Ala Leu Trp Ala Ala
   530 535 540
   Thr Phe Met Glu His Trp Lys Arg Lys Gln Met Arg Leu Asn Tyr Arg
   545 550 555 560
   Trp Asp Leu Thr Gly Phe Glu Glu Glu Glu Glu Ala Val Lys Asp His
   565 570 575
   Pro Arg Ala Glu Tyr Glu Ala Arg Val Leu Glu Lys Ser Leu Arg Lys
   580 585 590
   Glu Ser Arg Asn Lys Glu Thr Asp Lys Val Lys Leu Thr Trp Arg Asp
   595 600 605
   Arg Phe Pro Ala Tyr Phe Thr Asn Leu Val Ser Ile Ile Phe Met Ile
   610 615 620
   Ala Val Thr Phe Ala Ile Val Leu Gly Val Ile Ile Tyr Arg Ile Ser
   625 630 635 640
   Thr Ala Ala Ala Leu Ala Met Asn Ser Ser Pro Ser Val Arg Ser Asn
   645 650 655
   Ile Arg Val Thr Val Thr Ala Thr Ala Val Ile Ile Asn Leu Val Val
   660 665 670
   Ile Ile Leu Leu Asp Glu Val Tyr Gly Cys Ile Ala Arg Trp Leu Thr
   675 680 685
   Lys Ile Glu Val Pro Lys Thr Glu Lys Ser Phe Glu Glu Arg Leu Thr
   690 695 700
   Phe Lys Ala Phe Leu Leu Lys Phe Val Asn Ser Tyr Thr Pro Ile Phe
   705 710 715 720
   Tyr Val Ala Phe Phe Lys Gly Arg Phe Val Gly Arg Pro Gly Asp Tyr
   725 730 735
   Val Tyr Ile Phe Arg Ser Phe Arg Met Glu Glu Cys Ala Pro Gly Gly
   740 745 750
   Cys Leu Met Glu Leu Cys Ile Gln Leu Ser Ile Ile Met Leu Gly Lys
   755 760 765
   Gln Leu Ile Gln Asn Asn Leu Phe Glu Ile Gly Ile Pro Lys Met Lys
   770 775 780
   Lys Phe Ile Arg Tyr Leu Lys Leu Arg Arg Gln Ser Pro Ser Asp Arg
   785 790 795 800
   Glu Glu Tyr Ile Lys Arg Lys Gln Arg Tyr Glu Val Asp Phe Asn Leu
   805 810 815
   Glu Pro Phe Ala Gly Leu Thr Pro Glu Tyr Met Glu Met Ile Ile Gln
   820 825 830
   Phe Gly Phe Val Thr Leu Phe Val Ala Ser Phe Pro Leu Ala Pro Leu
   835 840 845
   Phe Ala Leu Leu Asn Asn Ile Ile Glu Ile Arg Leu Asp Ala Lys Lys
   850 855 860
   Phe Val Thr Glu Leu Arg Arg Pro Val Ala Ile Arg Ala Lys Asp Ile
   865 870 875 880
   Gly Ile Trp Tyr Asn Ile Leu Arg Gly Val Gly Lys Leu Ala Val Ile
   885 890 895
   Ile Asn Ala Phe Val Ile Ser Phe Thr Ser Asp Phe Ile Pro Arg Leu
   900 905 910
   Val Tyr Leu Tyr Met Tyr Ser Gln Asn Gly Thr Met His Gly Phe Val
   915 920 925
   Asn His Thr Leu Ser Ser Phe Asn Val Ser Asp Phe Gln Asn Gly Thr
   930 935 940
   Ala Pro Asn Asp Pro Leu Asp Leu Gly Tyr Glu Val Gln Ile Cys Arg
   945 950 955 960
   Tyr Lys Asp Tyr Arg Glu Pro Pro Trp Ser Glu His Lys Tyr Asp Ile
   965 970 975
   Ser Lys Asp Phe Trp Ala Val Leu Ala Ala Arg Leu Ala Phe Val Ile
   980 985 990
   Val Phe Gln Asn Leu Val Met Phe Met Ser Asp Phe Val Asp Trp Val
   995 1000 1005
   Ile Pro Asp Ile Pro Lys Asp Ile Ser Gln Gln Ile His Lys Glu Lys
   1010 1015 1020
   Val Leu Met Val Glu Leu Phe Met Arg Glu Glu Gln Gly Lys Gln Gln
   1025 1030 1035 1040
   Leu Leu Asp Thr Trp Met Glu Lys Glu Lys Pro Arg Asp Val Pro Cys
   1045 1050 1055
   Asn Asn His Ser Pro Thr Ala His Pro Glu Ala Gly Asp Ser Ser Pro
   1060 1065 1070
   Val Pro Ser Tyr Glu Tyr His Gly Gly Ala Leu
   1075 1080
<210> 6
   <211> 33
   <212> DNA
   <213> Artificial Sequence
<220>
   <223> Forward primer for cloning mouse AN01
<400> 6
   ccgctcgagg ccaccatgag ggtccccgag aag 33
<210> 7
   <211> 30
   <212> DNA
   <213> Artificial Sequence
<220>
   <223> Reverse primer for cloning mouse AN01
<400> 7
   ggggtaccct acagcgcgtc cccatggtac 30
<210> 8
   <211> 35
   <212> DNA
   <213> Artificial Sequence
<220>
   <223> Forward primer for cloning human AN01
<400> 8
   ctcgaggcca ccatgagggt caacgagaag tactc 35
<210> 9
   <211> 28
   <212> DNA
   <213> Artificial Sequence
<220>
   <223> Reverse primer for cloning human AN01
<400> 9
   ggtaccctac aggacgcccc cgtggtag 28
<210> 10
   <211> 37
   <212> DNA
   <213> Artificial Sequence
<220>
   <223> Forward primer for cloning human endothelin receptor type A
<400> 10
   gcggccgcgc cgccaccatg gaaacccttt gcctcag 37
<210> 11
   <211> 29
   <212> DNA
   <213> Artificial Sequence
<220>
   <223> Reverse primer for cloning human endothel in receptor type A
<400> 11
   tctctagatc agttcatgct gtccttatg 29
<210> 12
   <211> 33
   <212> DNA
   <213> Artificial Sequence
<220>
   <223> Forward primer for cloning rat histamine receptor type 1
<400> 12
   cgggatccgc caccatgagc tttgccaata cct 33
<210> 13
   <211> 45
   <212> DNA
   <213> Artificial Sequence
<220>
   <223> Reverse primer for cloning rat histamine receptor type 1
<400> 13
   ggaattctta ggaacgaatg tgcagaatct ttttgaatgt cttct 45
<210> 14
   <211> 33
   <212> DNA
   <213> Artificial Sequence
<220>
   <223> Forward primer for cloning human purinergic receptor P2Y, G-protein coupled 2
<400> 14
   cggaattcgc caccatggca gcagacctgg gcc 33
<210> 15
   <211> 33
   <212> DNA
   <213> Artificial Sequence
<220>
   <223> Reverse primer for cloning human purinergic receptor P2Y, G-protein coupled 2
<400> 15
   cgggatccct acagccgaat gtccttagtg ttc 33
<210> 16
   <211> 21
   <212> DNA
   <213> Artificial Sequence
<220>
   <223> Forward primer for RTQ-PCR amplification of mouse AN01
<400> 16
   tggcatttgt cattgtcttc c 21
<210> 17
   <211> 20
   <212> DNA
   <213> Artificial Sequence
<220>
   <223> Reverse primer for RTQ-PCR amplification of mouse AN01
<400> 17
   tcacccagtc cacaaagtca 20

## Description

### FIELD OF THE INVENTION

The present invention relates to a method of identifying agents which modulates the activity of a calcium-activated chloride channel protein, which is represented by the amino acid sequence of SEQ ID NO: 1.

### BACKGROUND OF THE INVENTION

Transport epithelia are thin epithelial sheets that cover glands, ducts, blood vessels, intestines, and airways, and allow the secretion and absorption of large quantities of water and electrolytes, which are fundamentally required to maintain water and electrolyte homeostasis and to moisten epithelia. Moreover, vectorial electrolyte transport provides a means transporting electrolytes across the epithelia. This form of transport involves the unidirectional movement of ions under the control of various ion transporters, pumps, and channels that are expressed differentially in the basolateral or apical membranes of epithelial cells (Hille, B., Ion Channels of Excitable Membranes (ed. Hille, B.) 269-306 (Sinauer Associates Inc, Sunderland, 2001)). Moreover, it is because of the physiological significance of vectorial transport in secretory and absorptive epithelia that channelopathies in epithelial layers often causes fatal diseases, such as, cystic fibrosis (CF) or pancreatic insufficiency (Eggermont, J., Proc Am Thorac Soc. 1, 22-7 (2004); Nilius, B. & Droogmans, G., Acta Physiol Scand 177, 119-47 (2003); and Rosenfeld, M. A. & Collins, F. S., Chest 109, 241-52 (1996)).

Chloride channels play fundamental roles in many physiological functions, and one group of these channels are activated by intracellular Ca²⁺, and accordingly, are collectively referred to as Ca²⁺-activated chloride channels (CaCCs). CaCCs control the apical outflux of Cl⁻, which is essential for the vectorial transport of electrolytes and water via secretory epithelia in kidneys, airways, intestine, pancreas, and salivary and lacrimal glands (Eggermont, J., Proc Am Thorac Soc. 1, 22-7 (2004); Nilius, B. & Droogmans, G., Acta Physiol Scand 177, 119-47 (2003); Hartzell, C. et al., J., Annu Rev Physiol 67, 719-58 (2005); and Kidd, J. F. & Thorn, P., Annu Rev Physiol 62, 493-513 (2000)). In addition, CaCCs are also known to contribute to cardiovascular functions by controlling vascular smooth muscle tone (Large, W. A. & Wang, Q., Am J Physiol 271, C435-54 (1996)) and by regulating cardiac myocyte excitability (Zygmunt, A. C., Calcium-activated Chloride Channels (ed. Fuller, C. M.) 81-98 (Academic Press, Amsterdam, 2002)). Moreover, by changing membrane potentials, CaCCs also regulate neuronal cell excitability, which is responsible for phototransduction, olfaction, gustation, and somesthetic sensations (Frings, S. et al., Prog Neurobiol 60, 247-89 (2000)).

CaCCs are known to mediate wide range of physiological functions. When stimulated by histamine, bradykinin or ATP, CaCCs facilitate the transport of Cl⁻ across the apical membranes of airway epithelia, whereas in exocrine glands (e.g., salivary glands or pancreas), CaCC agonists induce the secretion of fluid or electrolytes at the apical poles of acinar or ductal cells, where the transepithelial movement of Cl- under the control of CaCCs drives fluid movement (Hartzell, C. et al., J., Annu Rev Physiol 67, 719-58 (2005); Kidd, J. F. & Thorn, P., Annu Rev Physiol 62, 493-513 (2000); and Melvin, J. E. et al., Annu Rev Physiol 67, 445-69 (2005)). In renal tubules, CaCC activity is required for tubular reabsorption or Cl⁻ secretion (Kose, H. et al., Br J Pharmacol 131, 1689-99 (2000); and Boese, S. H. et al., J Physiol 523, 325-38 (2000)). In vascular smooth muscles, endothelin, norepinephrine, angiotensin II, and ATP evoke Ca²⁺-activated Cl⁻ conductance and thus powerful depolarization that induces contraction (Large, W. A. & Wang, Q., Am J Physiol 271, C435-54. (1996)). In olfactory receptor neurons, CaCCs amplify odorant-activated currents (Lowe, G. & Gold, G. H., Nature 366, 283-6 (1993); and Reisert, J. et al., J Gen Physiol 122, 349-63 (2003)), and in the mammalian retina, CaCCs probably participate in the regulation of lateral inhibition, a component of the visual signaling process (Barnes, S. & Deschenes, M. C., J Neurophysiol. 68, 745-55 (1992); and Maricq, A. V. & Korenbrot, J. I., Neuron 1, 503-15 (1988)). Moreover, in neuronal cells, like DRG neurons, CaCCs are responsible for after-depolarization (Frings, S., Reuter, D. & Kleene, S. J., Prog Neurobiol 60, 247-89 (2000)).

The above CaCC-mediated cellular responses are initiated via the stimulation of the G-protein coupled receptors (GPCRs) of hormones, paracrines, or neurotransmitters. Treatments with ATP, carbachol, endothelin-1, lysophosphatidic acid (LPA), histamine, and many other ligands evoke Cl⁻currents (Kajioka, S. et al., Am J Physiol Renal Physiol 286, F77-85 (2004); Nilius, B. et al., J Physiol. 498, 381-96 (1997); and Zholos, A. et al., J Gen Physiol 125, 197-211 (2005)). Ca²⁺-activated Cl⁻ currents are also activated by interventions that increase intracellular Ca²⁺, such as dialyzing Ca²⁺ into recording pipettes, injecting inositol 1,4,5,-triphosphate (IP₃), or directly applying Ca²⁺ to isolated membrane patches (Kuruma, A. & Hartzell, H. C., J Gen Physiol 115, 59-80 (2000); and Piper, A. S. & Large, W. A., J Physiol 547, 181-96 (2003)). Moreover, even though they are present in various tissue types, endogenous CaCCs in different cells retain characteristic biophysical and pharmacological properties, i.e., Ca²⁺-activated Cl⁻ currents are outwardly-rectifying, sensitive to Cl⁻ channel blockers, and their ion permeabilities follow the sequence I⁻ > Br⁻ > Cl⁻ (Eggermont, J., Proc Am Thorac Soc. 1, 22-7 (2004); Nilius, B. & Droogmans, G., Acta Physiol Scand 177, 119-47 (2003); Hartzell, C. et al., J., Annu Rev Physiol 67, 719-58 (2005); Kidd, J. F. & Thorn, P., Annu Rev Physiol 62, 493-513 (2000); and Frings, S. et al., Prog Neurobiol 60, 247-89 (2000)).

Meanwhile, cystic fibrosis (CF) is a life-threatening disease, in which Cl⁻ secretion by airway and other secretory epithelia is much reduced (Rosenfeld, M. A. & Collins, F. S., Chest 109, 241-52 (1996)). Cystic fibrosis transmembrane regulator (CFTR) was cloned by positional cloning technique for a gene responsible for the dysfunction in Cl⁻ secretion in these tissues (Rosenfeld, M. A. & Collins, F. S., Chest 109, 241-52 (1996); and Schwiebert, E. M. et al., Physiol Rev 79, S145-66 (1999)). CFTR is a Cl⁻ channel that is activated by intracellular c-AMP, and is known to be the primary Cl⁻ secreting channel in secretory epithelia. However, compelling evidence suggests that CaCCs are also involved in Cl⁻ secretion in these tissues. For example, activity of CaCCs was reported in CFTR deficient mice (Clarke, L. L. et al., Proc Natl Acad Sci U S A 91, 479-83 (1994); and Clarke, L. L. et al., Science 257, 1125-8 (1992)), which showed no reduction in fluid secretion or apparent CF symptoms (Grubb, B. R. et al., Am J Physiol 266, C1478-83 (1994)). Thus, in mice, CaCCs appear to compensate for CFTR loss of function. In contrast, such functional compensation is not evident in human CF, because CFTR dysfunction alone is sufficient to induce a CF-like pathology. However, evidence indicates the activation of latent CaCCs could compensate for CFTR loss of function in man. For example, Ca²⁺ ionophores effectively induced Ca²⁺-dependent Cl⁻ secretion in CF patients (Boucher, R. C. et al., J Clin Invest 84, 1424-31 (1989); and Anderson, M. P. & Welsh, M. J., Proc Natl Acad Sci U S A 88, 6003-7 (1991)). Furthermore, the treatment of human airway epithelial cells with UTP, a purinergic receptor agonist, was found to cause Cl⁻conductance in CF epithelia (Knowles, M. R. et al., N Engl J Med 325, 533-8 (1991); and Willumsen, N. J. & Boucher, R. C., Am J Physiol 256, C226-33 (1989)). Moreover, there appears to be a constitutive inverse relation between CFTR and CaCCs, i.e., CaCCs are downregulated by CFTR overexpression and upregulated by CFTR dysfunction (Kunzelmann, K. et al., Pflugers Arch 435, 178-81 (1997); and Wei, L. et al., Pflugers Arch 442, 280-5 (2001)). The inductions of Ca²⁺-activated Cl⁻ currents by Ca²⁺ ionophores or agonists are augmented in CF airways (Clarke, L. L. et al., Proc Natl Acad Sci U S A 91, 479-83 (1994); Grubb, B. R. et al., Am J Physiol 266, C1478-83 (1994); and Thomas, E. J. et al., Am J Physiol Cell Physiol 279, C1578-86 (2000)). These results inescapably suggest that CaCC activation could compensate for CFTR loss of function in CF.

Because of their pathophysiological significances, many efforts have been made to clone CaCCs, but the molecular identity that confers Ca²⁺-activated Cl⁻ currents has yet to be determined (Eggermont, J., Proc Am Thorac Soc. 1, 22-7 (2004); Nilius, B. & Droogmans, G., Acta Physiol Scand 177, 119-47 (2003); and Hartzell, C. et al., J., Annu Rev Physiol 67, 719-58 (2005)), which limits the understanding of the physiological functions and pathological conditions associated with CaCCs.

Thus, in order to obtain greater insight of the pathophysiological roles of CaCC, there exists a need to clone a Cl⁻ channel activated by intracellular Ca²⁺. Further, a specific need in the art exists for identification of agents capable of modulating calcium-dependent chloride secretion in order to develop a medicament for the treatment of diseases caused by the dysfunction of Ca²⁺-activated chloride channels.

### SUMMARY OF THE INVENTION

Accordingly, it is a major object of the present invention to provide a method of identifying an agent which modulates the activity of a calcium-activated chloride channel named anoctamin 1 (ANO1).

In accordance with one aspect of the present invention, there is provided an in vitro method of identifying an agent which modulates an activity of a protein, wherein the protein consists of the amino acid sequence of SEQ ID NO: 1 and transports chloride ions across a cell membrane, comprising: (a) exposing cells which express the protein to the agent; and (b) measuring degree of chloride ion transport in the exposed cells, wherein a change in the degree of chloride ion transport compared to control cells, which express the protein but not exposed to the agent, is indicative of an agent capable of modulating an activity of the protein.

In accordance with another aspect of the present invention, there is provided an in vitro method of identifying an agent which inhibits an activity of a protein, wherein the protein is represented by the amino acid sequence of SEQ ID NO: 1 and transports chloride ions across a cell membrane, comprising: (a) exposing cells that express the protein and a G-protein coupled receptor (GPCR) to the agent; (b) applying a ligand of the GPCR to the resulting cells; and (c) measuring degree of chloride ion transport in the exposed cells, wherein a reduction in the degree of chloride ion transport compared to control cells, which express the protein and GPCR but not exposed to the agent, is indicative of an agent capable of inhibiting the activity of the protein.

### BRIEF DESCRIPTION OF THE DRAWINGS

The above and other objects and features of the present invention will become apparent from the following description of the invention, when taken in conjunction with the accompanying drawings, which respectively show:
Figure 1: Multiple sequence alignments of mouse (SEQ ID NO: 1), human (SEQ ID NO: 3), and rat (SEQ ID NO: 5) anoctamin 1 (ANO1) sequences. Identical amino acids are marked in black. TM: transmembrane domain.
Figure 2: Predicted topology of ANO1 and its subunit composition.
   A. Phylogenetic tree depicting the anoctamin family in human (Tree View, http://taxonomy.zoology.gla.ac.uk/rod/treeview.html). Unit: 0.1 nucleotide substitutions per site.
   B. Predicted topology of mouse ANO1 (mANO1).
   C. Immunoblots of mANO1 and mANO1-GFP fusion protein using an anti-mANO1 antibody.
   D. ANO1 distribution in various organs.
   E. Localization of ANO1 mainly in the plasma membrane.

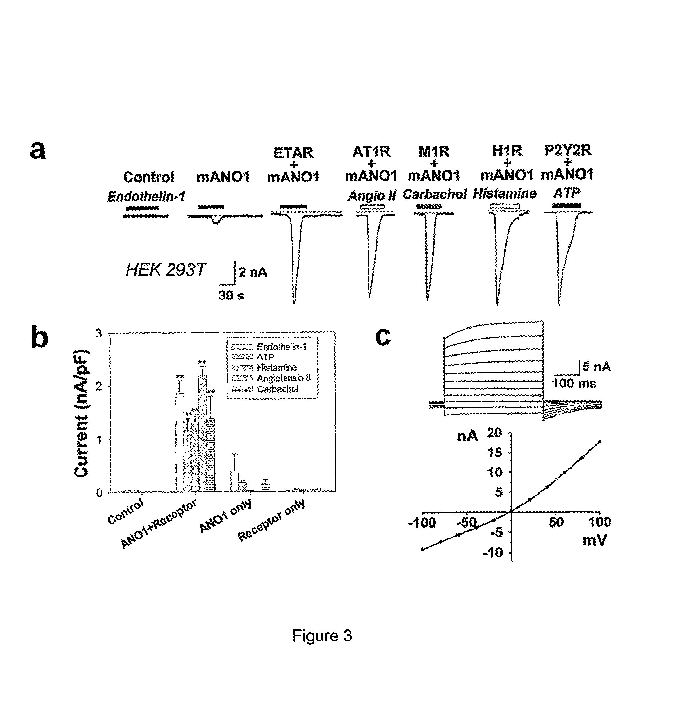

Figure 3: Robust inward currents evoked by mANO1 in response to G-protein coupled receptor (GPCR) stimulation, which is a physiological stimulus to CaCCs. mANO1 currents activated by GPCR stimulation elicits outwardly rectification, another hallmark of CaCC.
   A. Large inward currents were activated by endothelin-1 (50 nM), angiotensin II (1 µM, Angio II), carbachol (1 µM), histamine (100 µM), or ATP (100 µM) when mANO1 was co-transfected into HEK 293T cells with ET_{A}R, AT1R, M1R, H1R, or P2Y2R. E_{hold} = -60 mV. Dotted lines represent zero current.
   B. Summary of receptor stimulation activated currents (experiment numbers = 5-18). Some current responses of control, ANO1-transfected, and GPCR-transfected cells were too small to be seen. ** p < 0.001, compared with those of control cells or GPCR-transfected cells (ANOVA, Duncan's post hoc test).
   C. Current responses to voltage-steps ranging from -100 to +100 mV in 20 mV increments applied to HEK cells transfected with mANO1 and ET_{A}R. Current responses to voltage steps were recorded at peak currents evoked by 50 nM endothelin-1 (upper trace). Current-voltage relationship of currents in response to voltage step. Note that the current-voltage relationship shows outward rectification, a hallmark of endogenous CaCC.

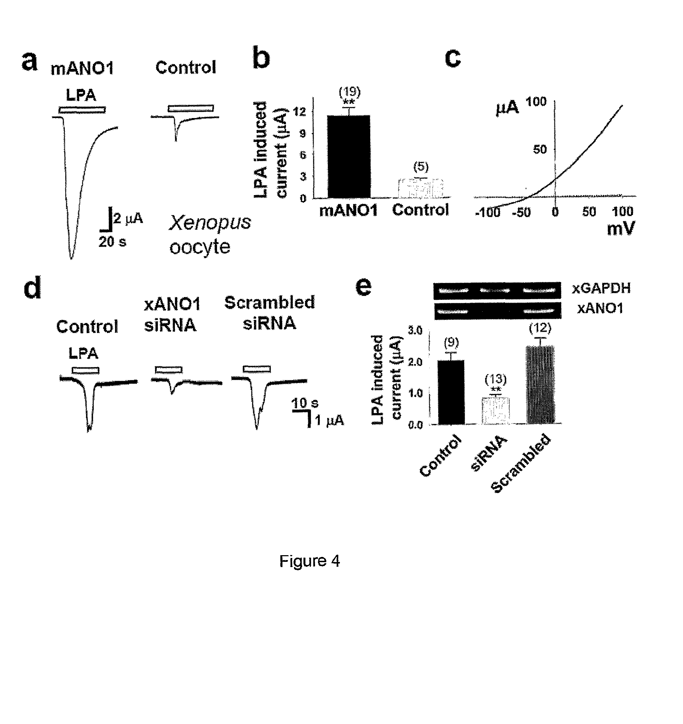

Figure 4: Ca²⁺-activated Cl⁻ currents conferred by ANO1 in *Xenopus* oocytes. *Xenopus* oocytes provide a recognized model for endogenous CaCC studies because they express endogenous CaCCs whose biophysical and pharmacological properties are similar to those of mammalian CaCCs. Thus, we expressed mANO1 in Xenopus oocytes and tested its activation by receptor stimulation using lysophosphatidic acid (LPA).
   A. Large inward currents evoked by the application of 1 µM LPA in *Xenopus* oocytes injected with mANO1 complimentary RNA, in contrast to small but robust currents evoked by LPA in water-injected oocytes (control). Holding potential: -60 mV. Bath solution: ND-96.
   B. Summary of LPA-evoked currents in mANO1 or water injected oocytes. ** p < 0.001.
   C. Current-voltage relationship of LPA-induced current. A voltage ramp from -100 to +100 mV was delivered to a *Xenopus* oocyte expressing mANO1 before (broken line) and after (black line) LPA application.
   D. Injecting siRNA of *Xenopus laevis* ANO1 (xANO1) reduced endogenous Ca²⁺-activated Cl⁻ current response to 1 µM LPA. LPA was applied to oocytes 5 days after water (control), siRNA, or scrambled siRNA injections.
   E. Summary of the effects of siRNA and scrambled siRNA on LPA-evoked currents. The levels of xANO1 transcripts in each group of oocytes were determined by RT-PCR after current recording. The level of Xenopus glyceraldehyde-3-phosphate dehydrogenase (xGAPDH) transcripts was determined for control. ** p < 0.001 compared to control (ANOVA, Duncan's post hoc test). Numbers in parenthesis are numbers of experiments. (Error bars = Mean ± S.E.M)

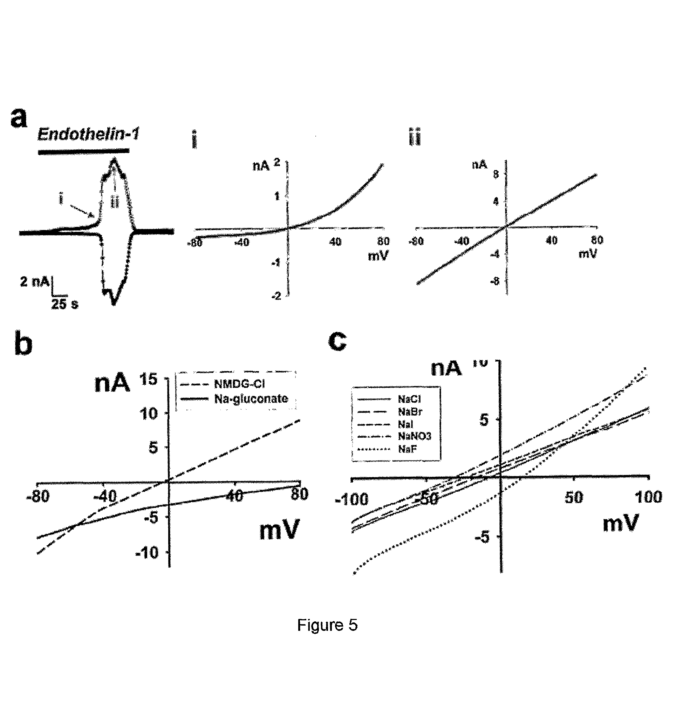

Figure 5: ANO1 is a Cl⁻ channel. To prove that mANO1 is a Cl⁻ channel, we expressed mANO1 in HEK cells and recorded its current evoked by endothelin-1 and performed various biophysical tests.
   A. Typical current-voltage relationships of endothelin-1 evoked currents at different time points in mAN01-ET_{A}R/HEK cells. Voltage ramps; -80 ∼ +80 mV, 320 ms duration, 0.83 Hz.
   B. Anion selectivity of endothelin-1 evoked currents in mANO1-ET_{A}R/HEK cells. Pipette solution contained 140 mM NMDG-Cl. Bath solutions contained 140 mM NMDG-Cl or Na-gluconate.
   C. Anion permeability. Bath solution contained 140 mM NaCl, NaBr, NaI, NaNO₃, or NaF.

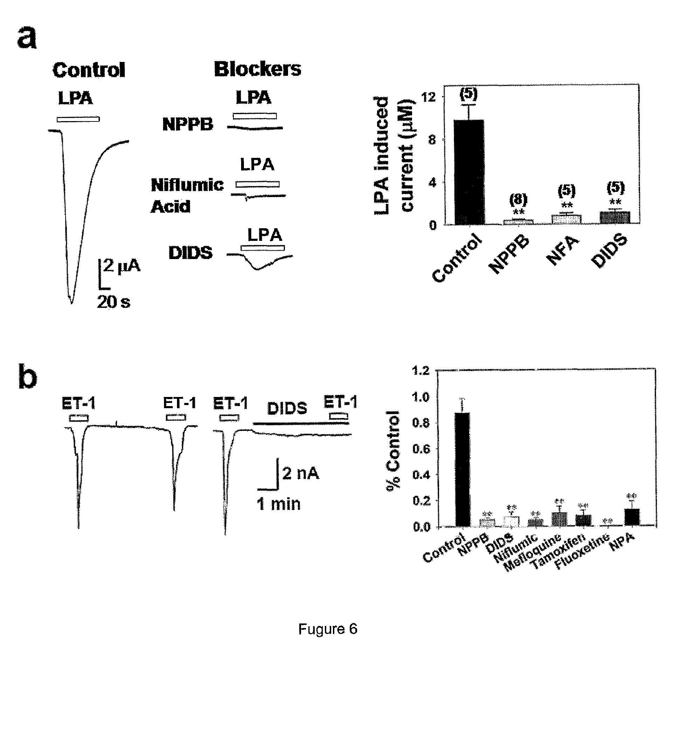

Figure 6: mANO1 current is blocked by specific pharmacological blockers for CaCCs as well as Cl⁻ channel blockers.
   A. (left) Inhibition of LPA-evoked currents in *Xenopus* oocytes injected with mANO1 RNA by pretreatment with the Cl⁻ channel blockers, 4,4'-diisothiocyanato-stilbene-2,2'-disulfonic acid (DIDS, 300 µM), niflumic acid (NFA, 200 µM), or 5-nitro-2-(3-phenyl-propylamino)-benzoate (NPPB, 200 µM). (right) Summary of the effects of Cl⁻ channel blockers on LPA-induced currents in oocytes. Numbers indicate the number of experiments. ** p < 0.001 versus currents in control oocytes (Student's T-test).
   B. Inhibition of endothelin-1 evoked currents in mANO1-ET_{A}R/HEK cells by Cl⁻ channel blockers as well as classical inhibitors (each, n = 5) of native CaCCs. Chemicals in 10 µM (except mefloquine, 5 µM) were applied 5 min before the second application of endothelin-1. Control (n=12). ** p < 0.001 compared to control (ANOVA, Duncan's post hoc test). (Error bars = Mean ± S.E.M)

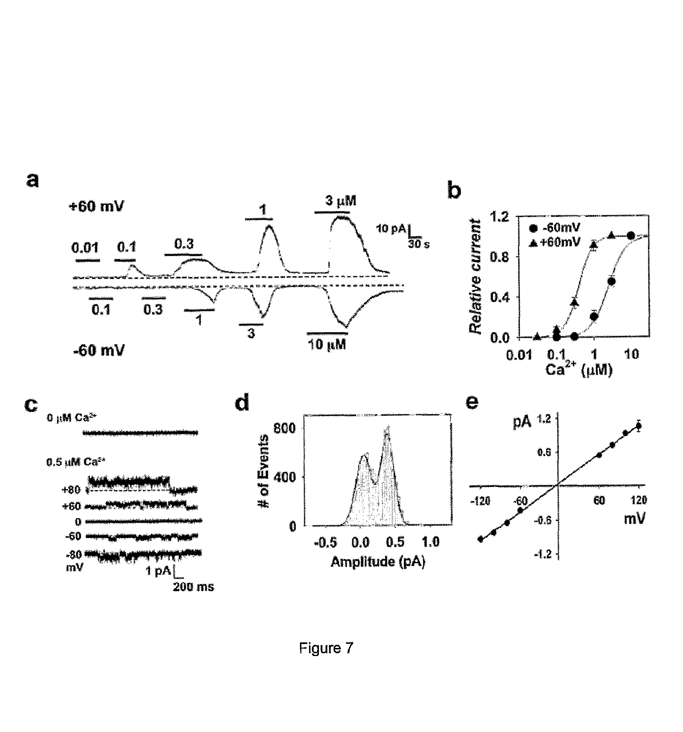

Figure 7: Activation of ANO1 by intracellular Ca²⁺ in a voltage-dependent manner.
   A. Macroscopic current responses to repeated applications of Ca²⁺ in various concentrations to the bath of inside-out patches isolated from mANO1-ET_{A}R/HEK cells at holding potentials of -60 or +60 mV. Pipette and bath solution contained 140 mM NMDG-Cl.
   B. Dose-response relationship of mANO1 activation by Ca²⁺. Current responses were normalized versus those observed at 10 µM (-60 mV, n = 8) or 3 µM Ca²⁺ (+60 mV, n = 8). Data points were fitted to the Hill's equation. (Error bars = Mean ± S.E.M)
   C. Single-channel currents activated by Ca²⁺ (0.5 µM) in an inside-out membrane patch. Signals were filtered at 2 kHz.
   D. An amplitude histogram of single channel currents activated by Ca²⁺ at +60 mV.
   E. Current-voltage relationship of single-channel Cl⁻ currents activated by Ca²⁺ (n = 3-7). Error bars (Mean ± S.E.M) are too small to be buried in symbols.

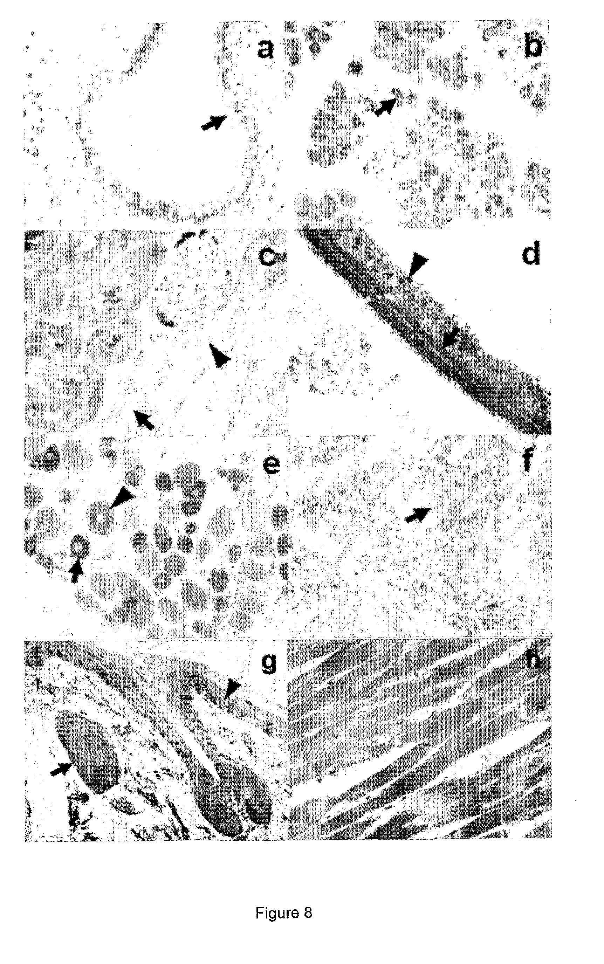

Figure 8: Expression of ANO1 in transport epithelia and other tissues.
   Residues 451 to 466 of mANO1 were chosen for generation of an ANO1 polyclonal antibody for immunohistochemical analysis. a: lung, b: pancreas, c: kidney, d: retina, e: dorsal-root ganglion, f: submandibular gland, g: skin, h: cardiac ventricle. Magnification = x400.

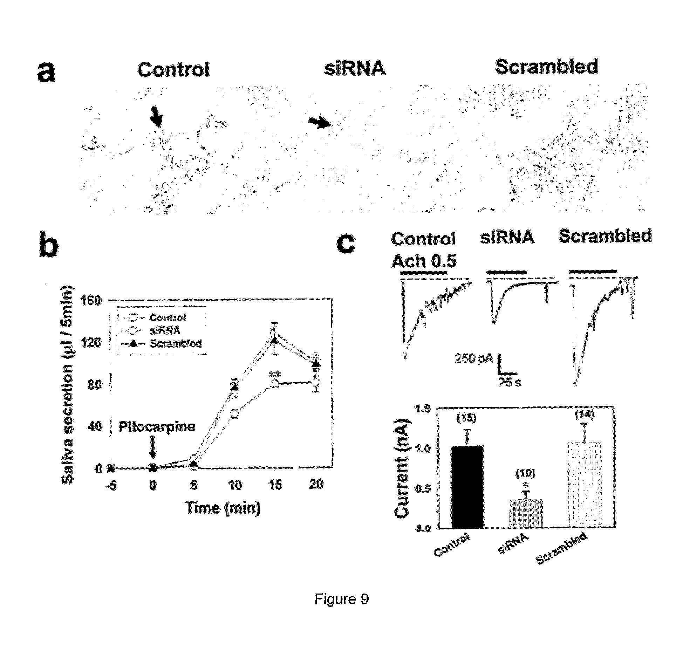

Figure 9: Down-regulation of mANO1 by mANO1 siRNA reduces pilocarpine-induced salivary output.
   A. Expression of mANO1 (arrow) in the submandibular glands of control, mANO1 siRNA-treated, and scrambled siRNA-treated animals.
   B. Effects of mANO1 siRNA treatment on saliva production. Saliva output was measured in every 5 min in control (n = 10), siRNA-treated (n = 8), and scrambled siRNA-treated (n = 10) mice. Pilocarpine (1 mg/kg) was injected intraperitoneally (arrow) to induce sufficient saliva production. ** p < 0.01 compared to control (ANOVA, Duncan's post hoc test). (Error bars = Mean ± S.E.M)
   C. Representative traces of acetylcholine (Ach, 0.5 µM)-induced currents in cells of submandibular glands isolated from mice injected with mANO1 siRNA or scrambled RNA four days before culture. (lower) Summary of current responses of acinar cells in submandibular glands. * p < 0.05 compared to control (ANOVA, Duncan's post hoc test). (Error bars = Mean ± S.E.M)

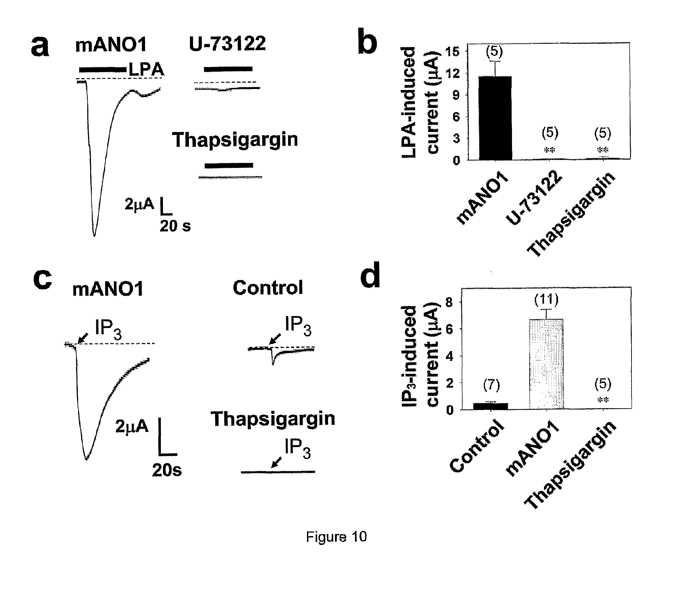

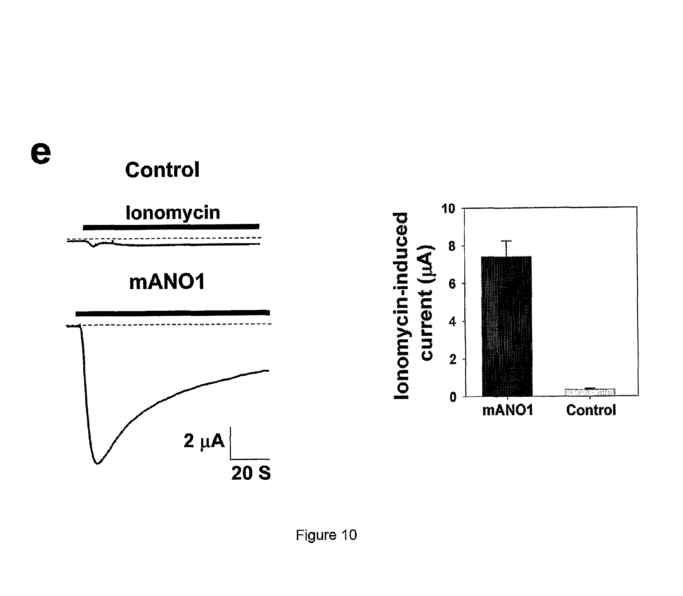

Figure 10: Blockage of ANO1 currents by a phospholipase C inhibitor (U-73122) and a Ca²⁺-depleting agent (thapsigargin) and activation thereof by injection of inositol-1,4,5-triphosphate (IP₃) to *Xenopus* oocytes expressing mANO1.
   A. Blockage of LPA-induced current responses by pretreatment with 1 µM thapsigargin or 10 µM U-73122 before LPA application.
   B. Summary of effects of thapsigargin or U-73122 pretreatment on LPA-induced ANO1 currents. Numbers in parenthesis are experimental numbers. ** p < 0.001 compared to untreated mANO1-injected oocytes.
   C. Injection of 2 mM IP₃ in 50 nl to oocytes expressing mANO1 evoked large inward currents (left panel) whereas the IP₃ injection induced small inward currents in water-injected oocytes (right upper). Pretreatment of 1 µM thapsigargin blocked the IP₃-induced current in a mANO1-expressing oocyte.
   D. Summary of effects of IP₃ injection to water (control)- or mANO1-injected oocytes and thapsigargin-pretreated mANO1 expressing oocytes. ** p < 0.001 compared to untreated mANO1-injected oocytes.
   E. Activation of mANO1 by a Ca²⁺ ionophore, ionomycin. Ionomycin (4 µM) was injected to control as well as mANO1 expressing oocytes. (Error bars = Mean ± S.E.M)

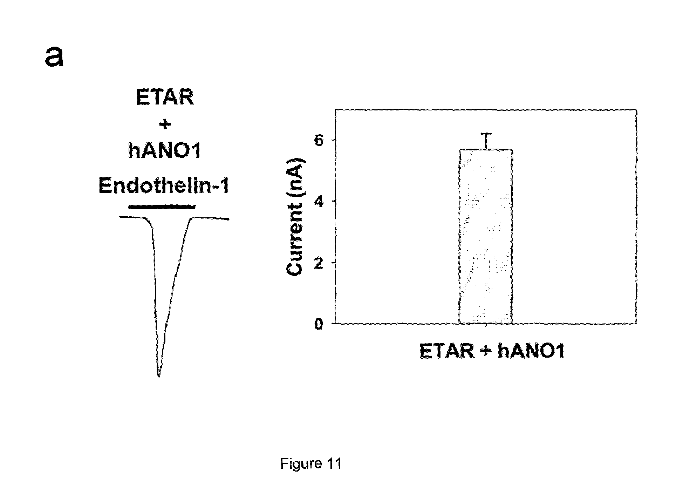

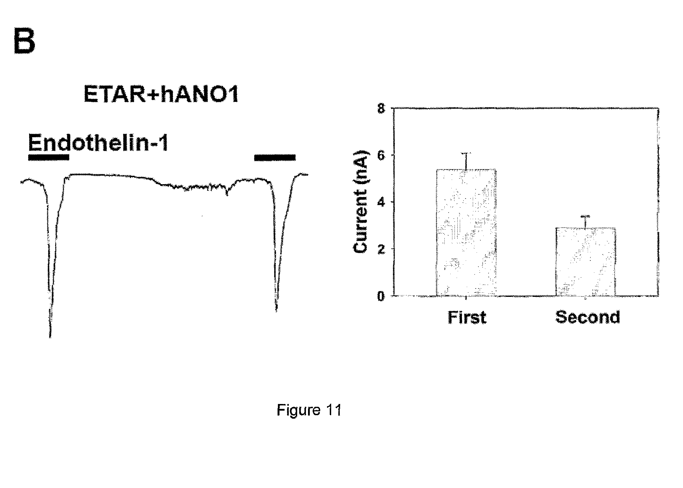

Figure 11: Human ANO1 (hANO1) is also activated by GPCR stimulation. hANO1 is cloned from human trachea and expressed in HEK cells along with ET_{A}R.
   A. Application of endothelin-1 (50 nM) to hANO1/ET_{A}R-HEK cells evoked large Cl⁻ currents. (right) Summary of hANO1 current evoked by endothelin-1. Pipette and bath solutions contained 140 mM NMDG-Cl.
   B. hANO1 responds to repeated stimulations by endothelin-1 and shows a small tachyphylaxis.

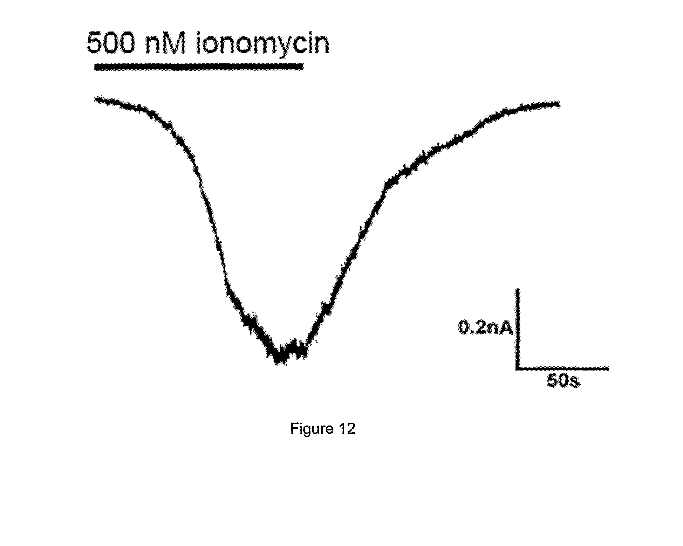

Figure 12: mANO1 stable cell line shows Cl⁻ currents in response to a Ca²⁺ ionophore, ionomycin. A stable cell line (HEK cells) that constitutively over-expresses mANO1 is constructed for high throughput screening analysis. In order to test whether the HEK cell line elicits ANO1 currents, ionomycin was applied. Application of ionomycin evoked a robust inward Cl⁻ currents in a whole cell. Pipette and bath solutions contained 140 mM NMDG-Cl.

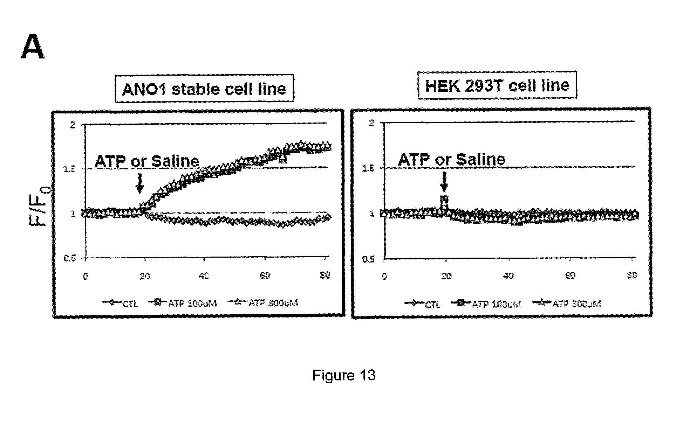

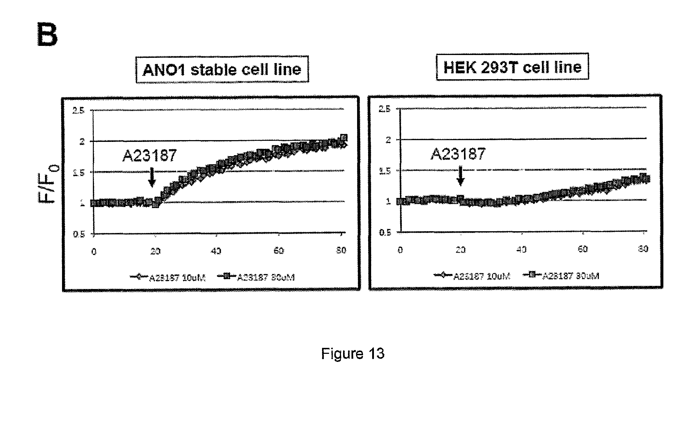

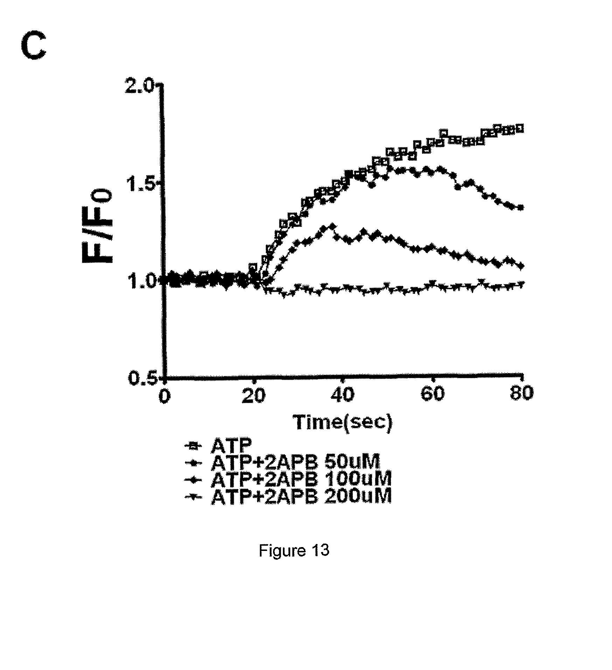

Figure 13: Result of a test for high-throughput screening (HTS) analysis. mANO1 stable cell line was grown in wells of a 96-well plate. mANO1-stably expressing HEK cells were incubated with a voltage sensor fluorescent dye (FLIPR^{®}-membrane potential assay kit, Molecular Device).
   A. After wash out the fluorescent dye, 100 µM ATP was applied. Application of ATP to mANO1-stably expressing HEK cells (left panel) evoked robust change in membrane potentials detected by fluorescent intensity, but not in control HEK cells (right panel). Fluorescent intensity was detected at excitation 530 nm and emission 565 nm by FlexStation 2 (Molecular device).
   B. Application of a Ca²⁺ ionophore, A23187, that increases intracellular Ca²⁺, evoked membrane potential change in ANO1-stably expressing HEK cells (left panel), but not in control HEK cells (right panel). F/F₀: ratio of fluorescence intensity.
   C. Pretreatment of 2APB, a known blocker of IP₃ receptor, blocked the ATP induced-membrane potential change in ANO1-stably expressing HEK cells.

### DETAILED DESCRIPTION OF THE INVENTION

The present invention includes a newly identified calcium-activated chloride channel(CaCC) proteins, which is represented by the amino acid sequence of SEQ ID NO: 1 and transports chloride ions across a cell membrane.

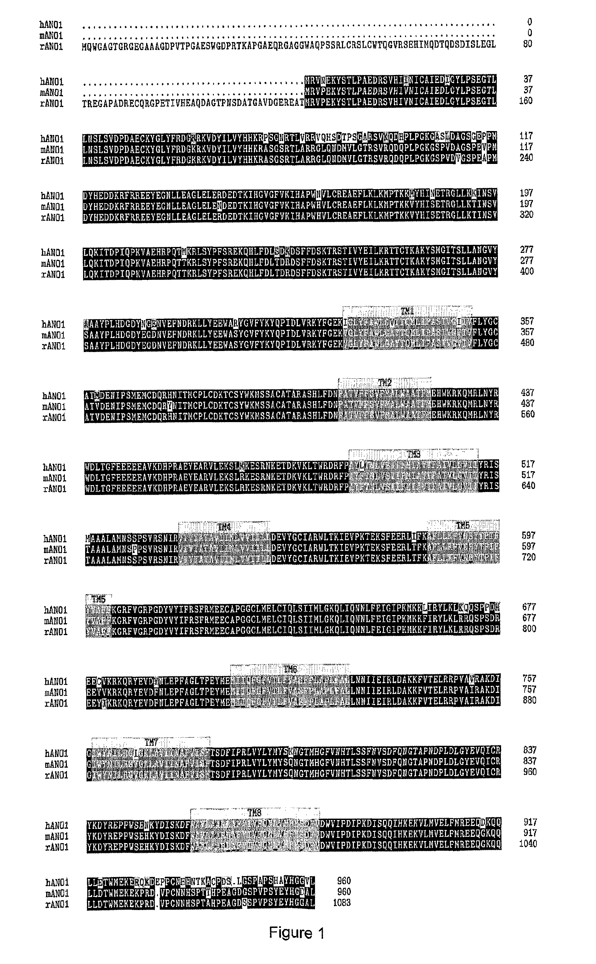

The proteins described herein include naturally occurring orthologs that have a slightly different amino acid sequence than that represented by SEQ ID NO: 1, which originates from a mouse. Exemplary orthologs comprises those from a human (SEQ ID NO: 3) and a rat (SEQ ID NO: 5) (Fig. 1).

The proteins further include conservative variants of the proteins herein described. As used herein, a conservative variant refers to alterations in the amino acid sequence that does not adversely affect the biological functions of the protein. A substitution, insertion or deletion is said to adversely affect the protein when the altered sequence prevents or disrupts a biological function associated with the protein. For example, the overall charge, structure or hydrophobic/hydrophilic properties of the protein may be altered without adversely affecting a biological activity. Accordingly, the amino acid sequence can be altered, for example to render the peptide more hydrophobic or hydrophilic, without adversely affecting the biological activities of the protein.

Ordinarily, the conservative variants will have an amino acid sequence having at least about 90 %, more preferably at least about 95 % amino acid sequence identity with the murine sequence set forth in SEQ ID NO: 1. Identity or homology with respect to such sequences is defined herein as the percentage of amino acid residues in the candidate sequence that are identical with the known peptides, after aligning the sequences and introducing gaps, if necessary, to achieve the maximum percent homology, and not considering any conservative substitutions as part of the sequence identity. N-terminal, C-terminal or internal extensions, deletions, or insertions into the peptide sequence shall not be construed as affecting homology.

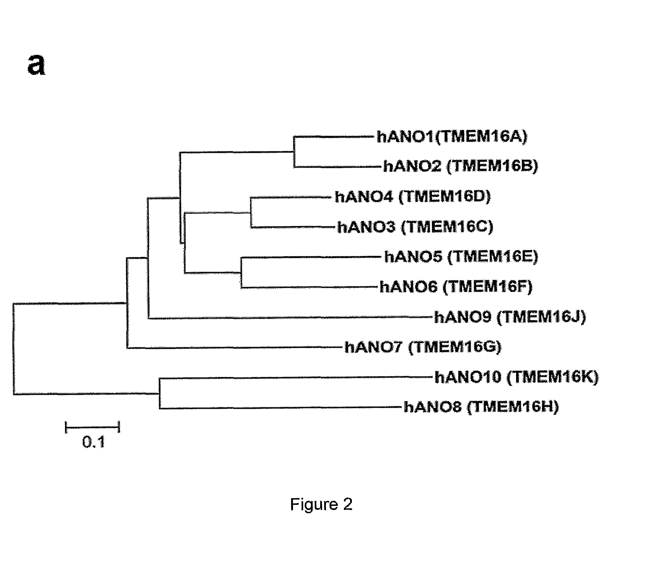

The gene for the CaCC of SEQ ID NO: 1 was identified by cDNA cloning experiments that were performed in order to identify new CaCC genes. Of the putative channel- or transporter-like genes whose functions had not been identified, TMEM16A attracted the present inventors' attention because it has multiple putative transmembrane domains and ten homologues in man (Fig. 2A). Because the expressed sequence tags of TMEM16A are frequently found in retina, full length complimentary DNA (cDNA) of mouse TMEM16A was amplified using reverse transcription-polymerase chain reaction (RT-PCR) from total RNA isolated from mouse eyes.

Mouse TMEM16A (SEQ ID NO: 1) has high homology (91 % identity in amino acid sequence) with its human ortholog (SEQ ID NO: 3; Fig. 1). Its predicted rat ortholog (SEQ ID NO: 5) has additional 123 amino acids at its N-terminus. The remainder of the rat sequence has high homology with mouse TMEM16A (99 % identity). TMEM16A is located in 11q13 human chromosome, where many tumor-related genes such as cyclin D1, fibroblast growth factor 3 and 4 precursor genes, myeloma overexpressed gene, and oral cancer overexpressed 1 gene, that are closely related to tumorigenesis, are clustered (Huang, X. et al., Genes Chromosomes Cancer 45, 1058-69 (2006) and Schwab, M., Bioessays 20, 473-9 (1998)). Interestingly, TMEM16A is highly amplified in head and neck skin cancer, oral cancer, and in other cancers.

Functionally-characterized native CaCCs have several common characteristics (Eggermont, J., Proc Am Thorac Soc. 1, 22-7 (2004); Hartzell, C. et al., J., Annu Rev Physiol 67, 719-58 (2005); Frings, S., Reuter, D. & Kleene, S. J., Prog Neurobiol 60, 247-89 (2000); Machaca, K. et al., Calcium-Activated Chloride Channels (ed. Fuller, C. M.) (Academic Press, Amsterdam, 2002); and Nilius, B. & Droogmans, G., Physiol Rev 81, 1415-59 (2001)). CaCCs are activated by the mobilization of intracellular Ca²⁺ when Ca²⁺ is directly applied to the patch or when Ca²⁺ is dialyzed into cells. This Ca²⁺ sensitivity is dependent on membrane potential, as CaCCs are more sensitive to Ca²⁺ at depolarization. CaCCs are permeable to monovalent anions in the order I⁻ > Br > Cl⁻, and are inhibited by various Cl⁻ channel blockers, e.g., DIDS, niflumic acid and NPPB. A small channel conductance is another character of CaCCs. More importantly, CaCCs are activated, via receptor stimulation, by a large class of ligands known to mobilize intracellular Ca²⁺. The cloned TMEM16A fulfills all of these biophysical as well as pharmacological profiles of native CaCCs.

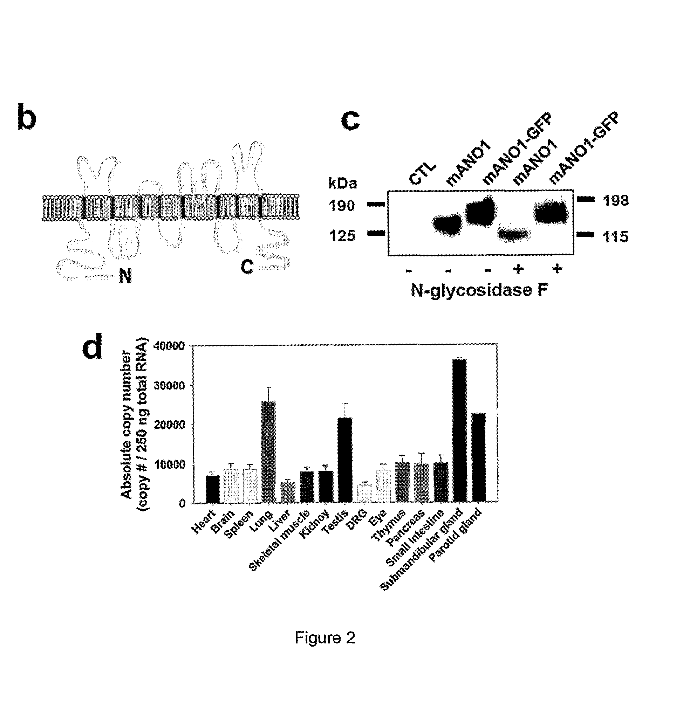

TMEM16A has eight transmembrane-spanning domains (Fig. 2B). Because TMEM16A has eight transmembrane domains and has anionic channel property, the present inventors designated it anoctamin 1 ("ANO1"). ANO1 has multiple protein kinase A, protein kinase C, protein kinase G, and casein kinase phosphorylation sites at intracellular protein segments and multiple glycosylation sites in extracellular segments. Even though ANO1 is activated by intracellular Ca²⁺, no specific consensus sites for Ca²⁺ binding, such as, the EF hand of calmodulin or the IQ motif for Ca²⁺-calmodulin as occurs in voltage-gated Ca²⁺ channels are present. Furthermore, no specific sequence homology is found between ANO1 and other cloned Cl⁻ channels, namely, cystic fibrosis conductance regulator (CFTR) Cl⁻ channel, ClC1, CLCA1, GABA receptor subtype 1, and glycine receptor subtype 1.

ANO1 is expressed in tissues in which CaCCs are known to be present, epithelia in airways, salivary glands, pancreatic ducts, renal tubules, intestines and skin express ANO1 at high to moderate levels. Moreover, dense ANO1 immunoreactivity is observed in all retinal layers and on sensory neurons, in which CaCCs are known to regulate photo- and sensory transduction (Frings, S., Reuter, D. & Kleene, S. J., Prog Neurobiol 60, 247-89 (2000)). Ventricular muscle express ANO1, which is consistent with the notion that ventricular action potentials are modulated by CaCCs (Zygmunt, A. C., Calcium-activated Chloride Channels (ed. Fuller, C. M.) 81-98 (Academic Press, Amsterdam, 2002)). The expression pattern of ANO1 clearly coincides with tissues that show CaCC activity, which further supports the notion that ANO1 is a candidate CaCC.

Also described herein are the nucleic acid molecules encoding the CaCCs, particularly, that encodes the protein of SEQ ID NO: 1 (ANO1) and that has at least 90 % nucleotide sequence identity thereto.

The nucleic acid molecules may include nucleic acid molecules that encode the proteins having SEQ ID NO: 3, SEQ ID NO: 5 and the related proteins herein described, preferably in isolated form. Exemplary nucleic acids are those represented by SEQ ID NOs: 2 and 4, which encodes the proteins of SEQ ID NOs: 1 and 3, respectively. As used herein, "nucleic acid" is defined as RNA or DNA that encodes a protein or peptide as defined above, or is complementary to nucleic acid sequence encoding such peptides, or encodes a polypeptide sharing at least 90 % sequence identity, more preferably at least 95 % sequence identity, with the peptide sequences.

As used herein, a nucleic acid molecule is said to be "isolated" when the nucleic acid molecule is substantially separated from contaminant nucleic acid encoding other polypeptides from the source of nucleic acid.

The present invention provides an in vitro method of identifying an agent which modulates an activity of a protein, wherein the protein is represented by the amino acid sequence of SEQ ID NO: 1 and transports chloride ions across a cell membrane, comprising: (a) exposing cells which express the protein to the agent; and (b) measuring degree of chloride ion transport in the exposed cells, wherein a change in the degree of chloride ion transport compared to control cells, which express the protein but not exposed to the agent, is indicative of an agent capable of modulating an activity of the protein.

The cells used in the inventive method may be prepared by transfecting a eukaryotic cell with a nucleic acid molecule that encodes the CaCC protein. Eukaryotic cells useful for the inventive method are not limited, so long as the cell line is compatible with cell culture methods and compatible with the expression of the gene product. Preferred eukaryotic host cells include, but are not limited to, mammalian cells, preferably vertebrate cells such as those from a mouse, rat, monkey or human cell line. The cells may be selected from the group consisting of epithelial cells, muscle cells, neuronal cells, and glandular cells. Preferred eukaryotic host cells include human embryonic kidney (HEK) cells, HEK 293 cells (ATCC CRL 1573), 3T3-L1 cells (ATCC CL 173), C6 cells (ATCC CCL 107), Chinese hamster ovary (CHO) cells (ATCC CCL61), CHO-K1 cells (ATCC CCL 61), NIH Swiss mouse embryo cells NIH/3T3 (ATCC CRL 1658), baby hamster kidney cells (BHK), COS-1 cells (ATCC CRL 1650), COS-7 cells (ATCC CRL 1651), HaCaT cells, HeLa cells (ATCC CCL 2), HeLa S3 cells (ATCC CCL 2.2), HepG2 cells (ATCC HB 8065), HL-60 cells (ATCC CCL 240), HUV-EC-C cells (ATCC CRL 1730), Jurkat cells (ATCC TIB 152), K-562 cells (ATCC CCL 243), L6 cells (ATCC CRL 1458), MCF7 cells (ATCC HTP 22), MDCK cells (ATCC CCL 34), NIH/3T3 cells (ATCC CRL 1658), RAW 264.7 cells (ATCC TIB 71), RBL-1 cells (ATCC CRL 1378), SH-SY5Y cells (ATCC CRL 2266), U-937 cells (ATCC CRL 1593.2) and the like eukaryotic tissue culture cell lines.

The identification of ANO1 has led to the discovery of compounds that are capable of up-regulating or down-regulating its activity. Activity is defined here as any alteration in either chloride channel function or expression of ANO1. Molecules that down-regulate ANO1 are therefore part of the invention. Down-regulation is defined here as a decrease in activation, function or synthesis of ANO1, its ligands or activators.

In the present invention, the agents that induce at least 10 percent, preferably at least 1,000 percent, change in the degree of chloride ion transport by the inventive CaCC, e.g., ANO1, as compared to that of the control cell without the agent, are selected as the desired agent. The change may be increase or decrease in the degree of chloride ion transport.

In the method of the present invention, degree of chloride ion transport may be determined by detecting electronic current responses as well as fluorescence changes that detect intracellular chloride concentration or changes in membrane potentials.

The inventive method may further comprises measuring expression of a specific protein, wherein expression of the specific protein is associated with the activity of the protein represented by the amino acid sequence of SEQ ID NO: 1.

The specific protein may be a salivary protein, lung protein, neuronal protein, pancreatic protein, heart protein, stomach and intestinal proteins, renal protein, retinal protein, and eccrine gland protein.

In accordance with another aspect of the present invention, there is disclosed an agent identified by said method.

The agent may be selected from the libraries of chemical compounds, natural products, oligonucleotides, peptides and antibodies.

The agents may be agonists or antagonists of ANO1.

Antagonists include those molecules that interact or bind to ANO1 and inactivate it. The present disclosure includes antagonists of ANO1 that block activation thereof. Antagonists are compounds that are themselves devoid of pharmacological activity but cause effects by preventing the action of an agonist. To identify an antagonist, one may test for competitive binding with natural ligands of ANO1. Assays of antagonistic binding and activity can be derived from monitoring ANO1 functions for down-regulation. The binding of the antagonist may involve all known types of interactions including ionic forces, hydrogen bonding, hydrophobic interactions, van der Waals forces and covalent bonds.

In addition, also disclosed herein are compounds that prevent the synthesis or reduce the biologic stability of ANO1.

Antagonists disclosed herein may be antibodies to ANO1. The antibodies to the ANO1 may be either monoclonal or polyclonal, made using standard techniques well known in the art. The antibodies are immunoreactive with critical positions of ANO1 protein. Antibody agents are obtained by the immunization of suitable mammalian subjects with peptides, containing as antigenic regions, those portions of the protein intended to be targeted by the antibodies.

Also described herein is a pharmaceutical composition comprising the agent modulating the activity of ANO1 or a protein having a sequence homology therewith, which were identified by the inventive method.

Further, described herein is a use of said agent for the manufacture of a medicament for treating a disease caused by the dysfunction of Ca²⁺-activated chloride channels.

Agents that modulate or down-regulate the expression of the protein or agents such as agonists or antagonists of at least one activity of the protein may be used to modulate biological and pathologic processes associated with the protein's function and activity. As used herein, a subject can be any mammal, so long as the mammal is in need of modulation of a pathological or biological process mediated by a protein described herein.

Pathological processes refer to a category of biological processes which produce a deleterious effect. Because CaCCs participate in a wide range of fundamental processes, it is conceivable that ANO1 dysfunction is likely to cause various diseases such as cystic fibrosis, xerostomia, xerophthalmia, hypertension, various types of cancers, renal diseases, arrhythmias, pancreatic disorders, and pain.

As used herein, an agent is said to modulate a pathological process when the agent reduces the degree or severity of the process. For instance, cystic fibrosis may be prevented or disease progression modulated by the administration of agents which reduce or modulate in some way the expression or at least one activity of ANO1.

Accordingly, the agents of the present disclosure may be used for the prevention or treatment of cystic fibrosis, xerostomia, xerophthalmia, hypertension, various types of cancers, renal diseases, arrhythmias, pancreatic disorders, pain, and various other chronic diseases.

Recently, compelling evidence has been presented indicating that ANO1/TMEM16A participates in tumorigenesis. TMEM16A was originally named TAOS2 (tumor amplified and overexpressed protein 2), because it is located in a locus on human chromosome 11q13, where genes such as cyclin D1, fibroblast growth factor 3 and 4 precursor genes are amplified in a wide variety of tumors, e.g., oral squamous cell carcinomas, esophageal, bladder, and breast cancers (Huang, X. et al., Genes Chromosomes Cancer 45, 1058-69 (2006); and Schwab, M., Bioessays 20, 473-9 (1998)). Gene microarray analysis revealed that ANO1/TMEM16A is upregulated -17 fold in squamous cell carcinoma of the head and neck (Carles, A. et al., Oncogene 25, 1821-31 (2006)), and that it is highly expressed in gastrointestinal stromal tumors (West, R. B. et al., Am J Pathol 165, 107-13 (2004)). The reason for this propensity of ANO1 in tumors is unknown, but it is conceivable that a secretory environment might be necessary for tumor cell proliferation. Therefore, the agents of the present disclosure may be used for the prevention or treatment of cancers in these tissues.

Xerostomia is dry mouth caused by medical treatments, various diseases, or aging. Xerostomia patients have reduced salivary flow and, therefore, they undergo difficulties in speech and swallowing, oral infections, and loss of appetite. Aged people now suffer from xerostomia. It is well known that CaCC play a critical role in salivary production (Arreola, J. et al., J Gen Physiol 108, 35-47 (1996)). Indeed, ANO1 immunoreactivity is highly present in salivary glands (Fig. 8F). Because ANO1 down-regulation by injection of ANO1 small interfering RNA causes reduction in salivary production (Fig. 9B), it is evident that ANO1 plays a critical role in producing saliva. Therefore, the agents of the present disclosure may be used for the prevention or treatment of xerostomia.

In a further aspect, the present inventors identify a disease state, which can be treated through the up-regulation of ANO1. Described herein is a method for use in treating the defect in cAMP-mediated chloride secretion in cystic fibrosis airway epithelia by further increasing calcium-dependent chloride secretion through up-regulation of ANO1. This up-regulation of ANO1 resulting in increased chloride secretion and thus restoration of the cellular chloride gradient resulting in normal airway epithelial cell function.

The agents of the present disclosure may be administered via parenteral, subcutaneous, intravenous, intramuscular, intraperitoneal, transdermal, or buccal routes. Alternatively, the agents may be administered by the oral route or directly to a target organ. The dosage will be dependent upon the age, health, and weight of the recipient, kind of concurrent treatment, if any, frequency of treatment, and the nature of the effect desired.

The agents may be administered systemically or topically, depending on such considerations as the condition to be treated, need for site-specific treatment, quantity of drug to be administered and similar considerations.

Topical administration may be used. Any common topical formation such as a solution, suspension, gel, ointment or salve and the like may be employed. Preparations of such topical formulations are well described in the art of pharmaceutical formulations. For topical application, these agents could also be administered as a powder or spray, particularly in aerosol form. The active ingredient may be administered in pharmaceutical compositions adapted for systemic administration. As is known, if a drug is to be administered systemically, it may be confected as a powder, pill, tablet or the like or as a syrup or elixir for oral administration. For intravenous, intraperitoneal or intralesional administration, the agent will be prepared as a solution or suspension capable of being administered by injection. In certain cases, it may be useful to formulate these agents in suppository form or as an extended release formulation for deposit under the skin or intramuscular injection. In a preferred aspect, the agents of this disclosure may be administered by inhalation. For inhalation therapy the agent may be in a solution useful for administration by metered dose inhalers or in a form suitable for a dry powder inhaler.

An effective amount is that amount which will down-regulate or up-regulate ANO1. A given effective amount will vary from condition to condition and in certain instances may vary with the severity of the condition being treated and the patient's susceptibility to treatment. Accordingly, a given effective amount will be best determined at the time and place through routine experimentation. However, it is anticipated that in the treatment of the diseases caused by ANO1 dysfunction, an amount between 0.01 and 500 mg/kg body weight/day, will usually constitute a therapeutically effective amount.

Also described herein are pharmaceutical compositions comprising the agents together with a pharmaceutically acceptable carrier. Pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. In addition to the pharmacologically active agent, the compositions may contain suitable pharmaceutically acceptable carriers comprising excipients and auxiliaries which facilitate processing of the active agents into preparations which can be used pharmaceutically for delivery to the site of action. Suitable formulations for parenteral administration include aqueous solutions of the active agents in water-soluble form, for example, water-soluble salts. In addition, suspensions of the active agents as appropriate oily injection suspensions may be administered. Suitable lipophilic solvents or vehicles include fatty oils, for example, sesame oil, or synthetic fatty acid esters, for instance, ethyl oleate or triglycerides. Aqueous injection suspensions may contain substances which increase the viscosity of the suspension include, for example, sodium carboxymethyl cellulose, sorbitol, and/or dextran. Optionally, the suspension may also contain stabilizers. Liposomes can also be used to encapsulate the agent for delivery into the cell.

The pharmaceutical formulation for systemic administration may be formulated for enteral, parenteral or topical administration, e.g., an aerosol formulation. Indeed, all three types of formulations may be used simultaneously to achieve systemic administration of the active ingredient.

Suitable formulations for oral administration include hard or soft gelatin capsules, pills, tablets, including coated tablets, elixirs, suspensions, syrups or inhalations and controlled release forms thereof.

In practicing the use or methods, the agents may be used alone or in combination, or in combination with other therapeutic or diagnostic agents. In certain aspects, the agents of may be co-administered along with other agents typically prescribed for the intended conditions according to generally accepted medical practice. The agents can be utilized *in vivo,* ordinarily in mammals, preferably in humans.

The following Examples are intended to further illustrate the present invention.

Further, percentages given below for solid in solid mixture, liquid in liquid, and solid in liquid are on a wt/wt, vol/vol and wt/vol basis, respectively, and all the reactions were carried out at room temperature, unless specifically indicated otherwise.

### Reference Example 1: Cloning of mouse and human ANO1

### (1) Mouse ANO1 cloning

First strand cDNAs were reverse transcribed from total RNA isolated from mouse eye using Power cDNA Synthesis Kit and oligo(dT) primer (iNtRON Biotech, Sungnam, Korea). Full-length coding sequence of mANO1 (TMEM16A; NM_178642.4 GI: 110835695) was obtained by RT-PCR using site-specific PCR primers; forward primer 5'-CCG CTC GAG GCC ACC ATG AGG GTC CCC GAG AAG-3' (SEQ ID NO: 6) and reverse primer 5'-GGG GTA CCC TAC AGC GCG TCC CCA TGG TAC-3' (SEQ ID NO: 7). The PCR product was inserted into pGEM-T easy vector (Promega, Madison, WI), cut with *Xho*I and *Kpn*I*,* and inserted into *Xho*I/*Kpn*I site of pSDTF (gift from Prof. Kang in Seoul National University) for oocyte expression or pEGFP-N1 (Clontech, Palo Alto, CA) for the expression of mANO1 in mammalian cells. The resulting vectors were designated pSDTF-mANO1 and pEGFP-N1-mANO1, respectively. pEGFP-N1 vector contains EGFP gene. In order to express an ANO1-EGFP fusion protein, a stop codon was deleted from pEGFP-N1-mANO1 using QuickChange site-directed mutagenesis kit (Stratagene, La Jolla, CA) because pEGFP-N1-mANO1 has a stop codon between the mANO1 and GFP coding sequences, and the resulting vector was designated pEGFP-N1-mANO1-GFP.

### (2) Human ANO1 cloning

The hANO1 (human TMEM16A; NM_018043, GI:40354209) was cloned from human trachea total RNAs (Clontech, Palo Alto, CA) by PCR method using following primers; forward primer 5'- CTC GAG GCC ACC ATG AGG GTC AAC GAG AAG TAC TC -3' (SEQ ID NO: 8) and reverse primer 5'- GGT ACC CTA CAG GAC GCC CCC GTG GTA G -3' (SEQ ID NO: 9). The PCR product was cut with *Xho*I and *Kpn*I*,* and then inserted into *Xho*I*lKpn*I site of pSDTF or pEGFP-N1.

### Reference Example 2: Cloning of GPCRs

Full-length cDNAs of human endothelin receptor type A (EDNRA(ET_{A}R); NM_001957, gi:4503464, Forward primer: 5'-GCG GCC GCG CCG CCA CCA TGG AAA CCC TTT GCC TCA G-3' (SEQ ID NO: 10), Reverse primer: 5'-TCT CTA GAT CAG TTC ATG CTG TCC TTA TG-3' (SEQ ID NO: 11)), rat histamine receptor type 1 (Hrh1; NM_017018, gi:7549757, Forward primer: 5'- CGG GAT CCG CCA CCA TGA GCT TTG CCA ATA CCT-3' (SEQ ID NO: 12), Reverse primer: 5'-GGA ATT CTT AGG AAC GAA TGT GCA GAA TCT TTT TGA ATG TCT TCT-3' (SEQ ID NO: 13)), and human purinergic receptor P2Y, G-protein coupled 2 (P2RY2 ; AY_136753, gi:22658488, Forward primer : 5'-CGG AAT TCG CCA CCA TGG CAG CAG ACC TGG GCC-3' (SEQ ID NO: 14), Reverse primer: 5'-CGG GAT CCC TAC AGC CGA ATG TCC TTA GTG TTC-3' (SEQ ID NO: 15)) were obtained by RT-PCR from single-strand cDNAs of HEK 293T cells, rat dorsal-root ganglion cells, and HaCaT cells, respectively.

Full-length cDNAs were inserted into mammalian expression vector pCDNA3.1 (Invitrogen), pEGFP-N1 (clontech) and pXOON (gift from Prof. T. Jespersen in University of Copenhagen), respectively. Rat angiotensin II receptor subtype 1 (Agtrla; NM_030985, GI:140969764) and human muscarinic receptor subtype 1 (CHRM1; NM_000738, GI:37622909) were donated by Dr. YS Bae (Ewha Women's University, Seoul, Korea) and Dr. CH Lee (Hanyang University, Seoul, Korea), respectively.

### Reference Example 3: Oocyte recording

pSDTF-mANO1 obtained in Reference Example 1 was linearized using *BamH*I*.* Complementary RNA was transcribed with SP6 RNA polymerase (mMESSAGE mMACHINE^{®} SP6 Kit, Ambion, Austin, TX). After being defolliculated with collagenase (Type IA, Sigma), *Xenopus laevis* oocytes were injected with 50 ng of the complementary RNAs of mANO1 in 50 nl of diethylpyrocarbonate-treated water. Two to five days after the injection, whole cell currents were recorded using the two-electrode voltage clamp technique. The bath solution contained 96 mM NaCl, 1 mM MgCl₂, 5 mM HEPES, 2 mM KCl and 1.8 mM CaCl₂ at pH 7.5 and was perfused at 2 ml/min. Experiments were performed at room temperature.

### Reference Example 4: RTQ-PCR Analysis

Real-time quantitative PCR (RTQ-PCR) was used to measure ANO1 expression levels in tissues. Mouse ANO1 specific universal probe (#9, 5'-TGG TGA TG-3') and PCR primers (Forward primer: 5'-TGG CAT TTG TCA TTG TCT TCC-3' (SEQ ID NO: 16), Reverse primer: 5'-TCA CCC AGT CCA CAA AGT CA-3' (SEQ ID NO: 17)) were designed using ProbeFinder software (http://www.universalprobelibrary.com). RTQ-PCR amplifications were performed using a LightCycler 2.0 system (Roche Applied Bioscience, Mannheim, Germany) according to the manufacturer's protocol. Absolute copy number per 250 ng of total RNA was calculated from crossing-point value with a standard curve using external standard method. The standard curve was set up with five samples, which were prepared by serial dilution of linearized plasmid containing mANO1 cDNA. The reactions were done in triplicate.

### Reference Example 5: Mammalian Cell Expression

HEK 293T cells were transfected with pEGFP-N1 (control) (1.0 µg) or pEGFP-N1-mANO1 (0.8∼1.0 µg) and/or any one of the GPCR cDNAs (1.0 µg) obtained in Reference Example 2, using a FuGENE^{®} HD transfection reagent (Roche Diagnostics, Penzberg, Germany). Transfected cells were maintained in DMEM supplemented with heat inactivated 10% fetal bovine serum (GIBCO BRL, Rockville, MD) and penicillin-streptomycin at 37□ in a 5 % CO₂ humidified atmosphere. One or two days after transfection, transfected cells were used for biochemical and electrophysiological experiments.

### Reference Example 6: Antibody production

Polyclonal antibody to mouse ANO1 was raised in a rabbit using synthetic peptide corresponding to the cytosolic region of ANO1 between TM2 and TM3 (451^{st}∼466^{th} amino acids of SEQ ID NO: 1: KDHPRAEYEARVLEKS) (AbFrontier Co., Seoul, Korea). Briefly, the peptide was synthesized by Thermo (Thermo Electron, Bremen, Germany) to immune grade. Rabbits were immunized by injecting subcutaneously into their backs 2.5 mg of the peptide antigen conjugated to 10 mg of keyhole limpet haemocyanin carrier protein emulsified in complete Freund's adjuvant. Four weeks after the first immunization, rabbits were boosted sequentially 3 times, at 2 weeks intervals, with peptide-keyhole limpet haemocyanin conjugate (500 µg) in incomplete Freund's adjuvant. Mouse ANO1 polyclonal antibody was purified by antigen peptide specific affinity column chromatography and protein A-Sepharose column chromatography. The specificity of purified antibody was confirmed by immunoblotting.

### Reference Example 7: Construction of stable cell line of ANO1

HEK293 cells were transfected with pEGFP-N1-mANO1 using Lipofectamine (Invitrogen Corp., Carlsbad, CA). After selection in 800 µg/ml geneticin (Invitrogen Corp., Carlsbad, CA) for 15 days, single colonies were picked with cloning cylinders and tested for activity of ANO1.

### Reference Example 8: Immunohistochemical staining of ANO1

Sections (4 µm thickness) of 10 % formalin-fixed, paraffin-embedded tissues were cut on silanized glass slides, deparaffinized three times with xylene, rehydrated in ethanol series, and then immersed in 0.3 % H₂O₂ to quench endogenous peroxidase activity. Sections were then microwaved in 10 mM citrate buffer (pH 6.0) for 20 min for antigen retrieval, incubated with 1:400 diluted rabbit anti-mouse ANO1 polyclonal antibody obtained in Reference Example 6 for 60 min, and rinsed three times with rinsing solution (distilled water containing 0.1 % Tween 20). They were then incubated with dextran polymer conjugated with goat anti-rabbit antibody and horseradish peroxidase (Envision plus kit, DAKO, Carpinteria, CA) for 20 minutes at room temperature, rinsed, and washed. The chromogen was then developed over 5 minutes with liquid 3, 3'-diaminobenzidine. The sections were then counter-stained with Meyer's hematoxylin, dehydrated, and mounted using Canada balsam. Negative controls with normal rabbit serum were processed in parallel; no positive staining was observed.

### Reference Example 9: Current recordings

To form gigaseals, individual cell surfaces were touched with a ~3 Mohm Sylgard-coated glass pipette with gentle suction, and the junction potential was then adjusted to 0 mV. The membrane in contact with the pipette was then ruptured by suction to form a whole cell. The pipette solution contained 140 mM NMDG-Cl, 2 mM MgCl₂, 10 mM HEPES, 2 mM ATP, and 0.3 mM GTP adjusted to pH 7.2, and the bath solution contained 140 mM NMDG-Cl, 2 mM MgCl₂ and 10 mM HEPES (pH 7.2). For ion selectivity experiments, 140 mM NMDG-Cl was replaced with equimolar NaCl, NaI, NaBr, NaNO₃, NaF, or Na-gluconate in the bath solution. To determine current-voltage relationships, voltage ramps from -80 or -100 mV to +80 or +100 mV were delivered for 350 ms, or voltage-pulses from -100 mV to +100 mV were delivered for 400 ms to whole cells. When mANO1 was activated by 300 nM intracellular Ca²⁺, voltage-pulses of 2500 ms were delivered. Whole-cell currents were amplified using an Axopatch 200B amplifier (Molecular Device, Sunnyvale, CA) and stored in a personal computer after digitization by a Digidata 1440 (Molecular Device). For single-channel current recordings, cell-attached or inside-out patches were formed from gigaseals. The pipette and control bath solution contained 140 mM NMDG-Cl, 2 mM MgCl₂ and 10 mM HEPES (pH 7.2). Currents were amplified, filtered at 2 kHz, and stored in a data recorder, and when required this data was exported to a personal computer for current amplitude analysis. To create amplitude histograms, pClamp software (version 6.0, Molecular Device) was used to analyze open and closed single-channel current events. The half-amplitude algorithm was used to determine an open event. The minimum duration of an open event was set at 0.1 ms.

### Reference Example 10: Measurement of activity of ANO1 by voltage-sensing fluorescence

The activity of the ANO1-stable cell line was characterized with a FLIPR^{®} Membrane Potential assay kit (Molecular devices, Sunnyvale, CA) according to the manufacture's instructions. ANO1-stable cell lines were seeded into poly-D-lysine coated 96-well plates (50,000 cells/ each well). One day after the plating, the cells were incubated with 50 µℓ Hank's balanced salt solution (HBSS) containing membrane potential dye for 40 min at 37 °C. To test the activity of ANO1 stable cell line, 100□µM ATP or 10□µM A23187 (Sigma, St Louis, MO, USA) was added to each well for 20 sec after starting detection. The fluorescence intensity was measured by a fluorometric imaging plate reader (FlexStation™ II, MolecularDevices). Membrane potential dye was excited at 530 nm, and the intensity of fluorescence emitted at 565 nm was measured.

### Example 1: Cloning and characterization of ANO1

### (1) Cloning of ANO1

To clone a putative CaCC, the present inventors searched public domain databases for putative channel- or transporter-like genes with more than two transmembrane domains and multiple isoforms. Of the putative channel- or transporter-like genes whose functions had not been identified, TMEM16A attracted our attention because it has multiple putative transmembrane domains and ten homologues in man (Fig. 2A). Because the expressed sequence tags of TMEM16A are frequently found in retina, full length TMEM16A complimentary DNA (cDNA) was amplified using RT-PCR (reverse transcription-polymerase chain reaction) from total RNA isolated from mouse eye and cloned in accordance with the method of Reference Example 1.

Mouse TMEM16A cDNA contains an open reading frame of 2,880 nucleotides (SEQ ID NO: 2) that encodes for a protein of 960 amino acids (SEQ ID NO: 1; Fig. 1). Cloned TMEM16A has 12 additional nucleotides, i.e., 1342-GGAAGCTGTCAA-1353, that encode four additional amino acids (448-EAVK-451), which are not included in the reported nucleotide sequence (NM_178642, gi:110835695) in the public domain, BLAST. Mouse TMEM16A has high homology (91 % amino-acid sequence identity) with its human ortholog (SEQ ID NO: 3; Fig. 1), and its predicted rat ortholog (SEQ ID NO: 5) has an additional 123 amino acids at its N terminus; the remainder of the rat sequence has high homology with mouse TMEM16A (99 % identity). TMEM16A is located in the 11q13 human chromosome, where many tumor-related genes such as cyclin D1, fibroblast growth factor 3 and 4 precursor genes, myeloma overexpressed gene, and oral cancer overexpressed 1 gene that are closely related to tumorigenesis are clustered (Huang, X. et al., Genes Chromosomes Cancer 45, 1058-69 (2006); and Schwab, M., Bioessays 20, 473-9 (1998)). Interestingly, TMEM16A is highly amplified in head and neck skin cancer, oral cancer, and in other cancers.

### (2) Hydropathy plot analysis

Hydropathy plot analysis (HMMTOP 2.0 software, http://www.enzim.hu/hmmtop) suggested that TMEM16A has eight transmembrane-spanning domains (Fig. 2B). Because TMEM16A has eight transmembrane domains and an anionic channel property (see below), the present inventors designated it anoctamin 1 (ANO1). Consensus analysis (Myhits motif scan software, http://myhits.isb-sib.ch/cgi-bin/motif_scan) predicted that ANO1 has multiple protein kinase A, protein kinase C, protein kinase G, and casein kinase phosphorylation sites at intracellular protein segments and multiple glycosylation sites in extracellular segments. Even though ANO1 is activated by intracellular Ca²⁺ (see below), no specific consensus sites for Ca²⁺ binding, such as, the EF hand of calmodulin or the IQ motif of Ca²⁺-calmodulin (as occurs in voltage-gated Ca²⁺ channels) were found. Furthermore, no specific sequence homology was found between ANO1 and other cloned Cl⁻ channels, namely, cystic fibrosis conductance regulator (CFTR) Cl⁻ channel, ClC1, CLCA1, GABA receptor subtype 1, and glycine receptor subtype 1.

### (3) Western blotting

For a Western blotting analysis, total lysates of HEK 293T cells transfected with pEGFP-N1-mANO1 or pEGFP-N1-mANO1-EGFP were collected, electrophoresed in 10% SDS-PAGE gels, transferred to PVDF membranes, and then blotted with anti-mANO1 polyclonal antibody (1:5,000). N-glycosidase F was added to cleave potential glycosylated adducts. Consequently, a ∼130 kDa protein was observed to migrate. This protein was slightly greater than expected for mANO1 (110 kDa), presumably because of glycosylation. However, when lysates were treated with N-glycosydase F, a discrete band of 110 kDa was observed (Fig. 2C).

### (4) Real-time quantitative PCR

Real-time quantitative PCR was performed to determine the levels of ANO1 transcripts in various organs, in accordance with the method of Reference Example 4. As shown in Fig. 2D, ANO1 transcripts were found to be distributed evenly throughout various organs, but much higher levels were found in lung and testes.

### (5) Intracellular localization of ANO1

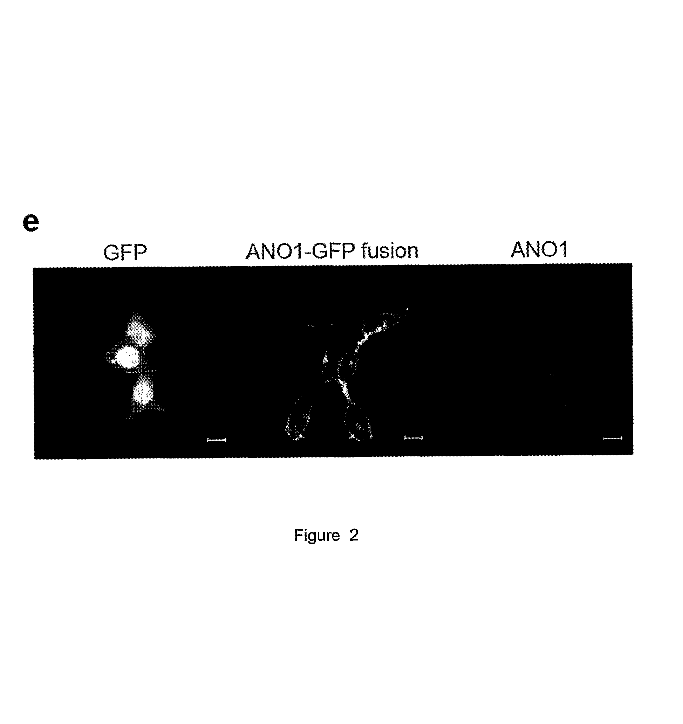

In order to determine intracellular localization of ANO1, HEK 293T cells were transfected with vector pEGFP-N1, pEGFP-N1-mANO1-GFP, or pEGFP-N1-mANO1 prepared in Reference Example 1 and confocal microscopic images were taken therefrom. mANO1 was visualized using mANO1 antibody and Alexa Fluor 594-conjugated secondary antibody (goat anti-rabbit IgG). When heterologously expressed in HEK 293T cells, the ANO1-GFP fusion protein was observed at the plasma membrane, whereas heterologously expressed GFP was expressed mainly in cytosol (Fig. 2E; pEGFP-N1 (left), pEGFP-N1-mANO1-GFP (middle), or pEGFP-N1-mANO1 (right)). mANO1 was found to localize to the plasma membrane of HEK 293T cells transfected with mANO1.

### Example 2: ANO1 is operated by receptor stimulation

Under physiological conditions CaCCs are known to be activated by GPCR stimulation. Endothelin-1 is a powerful vasoconstrictor that depolarizes vascular smooth muscle by activating CaCCs (Klockner, U. & Isenberg, G., Pflugers Arch. 418, 168-75 (1991); and Salter, K. J. & Kozlowski, R. Z., J Pharmacol Exp Ther. 279, 1053-62 (1996)). Thus, mANO1 and endothelin receptor subtype A (ET_{A}R) were transfected into HEK 293T cells, along with GFP for visual identification of transfected cells, in accordance with the method of Reference Example 5. Whole cells were used to test the current response of mANO1 to ET_{A}R stimulation. Pipette and bath solutions contained 140 mM N-methyl D-glucamine (NMDG)-Cl, which permitted Cl⁻ to be used as the main charge carrier. The pipette solution was Ca²⁺ free and contained no EGTA. Application of 50 nM endothelin-1 to mANO1-ET_{A}R/HEK cells evoked robust inward currents when the membrane potential was held at -60 mV. In contrast, endothelin-1 evoked currents were minimal in HEK cells transfected with ET_{A}R or control (GFP-transfected only) cells. When mANO1 was transfected alone, 50 nM endothelin-1 evoked appreciable currents; presumably because of the presence of endogenous endothelin receptors in HEK cells. Similarly, the co-expression of mANO1 in HEK cells with muscarinic receptor subtype 1 (M1R), histamine receptor subtype 1 (H1R), purinergic receptor subtype 2 (P2Y2R) or angiotensin receptor subtype 1 (AT1R) (all known to use CaCCs for their cellular functions (Zholos, A. et al., J Gen Physiol 125, 197-211 (2005); Guibert, C. et al., Am J Physiol 270, L637-42 (1996); and Wayman, C. P. et al., Br J Pharmacol 121, 1301-8 (1997)) resulted in robust current responses to carbachol (1 µM), histamine (100 µM), or angiotensin II (1 µM) (Figs. 3A and 3B). In contrast, these ligands evoked minimal currents when HEK cells were transfected with these receptors alone, or in control (GFP transfected only) cells. Moreover, in mANO1-transfected cells, the application of carbachol evoked small but significantly greater currents than in control cells, presumably because of the presence of endogenous receptors for these ligands in control HEK cells (Fig. 3B).

The current-voltage relationship of endothelin-1-induced ANO1 currents was weakly-outwardly rectifying or linear (Fig. 3C), which is consistent with the linear current-voltage relationships of endogenous CaCCs when intracellular Ca²⁺ concentrations are high (Fig. 5A) (Kuruma, A. & Hartzell, H. C., J Gen Physiol 115, 59-80 (2000); Piper, A. S. & Large, W. A., J Physiol 547, 181-96 (2003); and Evans, M. G. & Marty, A., J Physiol 378, 437-60 (1986)). Taken together, these results demonstrate that ANO1 is activated by receptor stimulation.

### Example 3: Activation of mANO1 in Xenopus oocytes

*Xenopus* oocytes provide a recognized model for endogenous CaCC studies because they express endogenous CaCCs that are required for 'fertilization depolarization' (Hartzell, H. C. et al., Mol Pharmacol 51, 683-92 (1997)). Furthermore, the biophysical and pharmacological properties of *Xenopus* CaCCs are similar to those of mammalian CaCCs (Machaca, K. et al., Calcium-Activated Chloride Channels (ed. Fuller, C. M.) (Academic Press, Amsterdam, 2002)). Accordingly, the present inventors expressed mANO1 in *Xenopus* oocytes and tested its activation by receptor stimulation using 1 µM LPA (lysophosphatidic acid). CaCCs in *Xenopus* are known to be activated by LPA (Guo, Z. et al., Proc Natl Acad Sci U S A. 93, 14367-72 (1996); and Kim, M. J. et al., Biochem Pharmacol. 59, 241-7 (2000)). LPA application to water-injected oocytes was found to cause inward currents comparable to those reported (Guo, Z. et al., *supra;* and Kim, M. J. et al., *supra)* (Figs. 4A and 4B). In contrast, when LPA was applied to *Xenopus* oocytes injected with complimentary mANO1 RNA in accordance with the method of Reference Example 3, current response to LPA increased about six fold (Fig. 4B). Moreover, LPA-induced ANO1 currents were outwardly-rectifying, like those of native CaCCs in *Xenopus* oocytes (Machaca, K. et al., *supra)* (Fig. 4C). The reversal potential was 37.7 ± 1.05 mV (n = 5), which is close to the equilibrium potential of Cl⁻ in *Xenopus* oocytes (Weber, W., Biochim Biophys Acta 1421, 213-33 (1999)).

### Example 4: Signaling pathway for receptor-operated CaCC

Various GPCRs are known to activate CaCCs via the action of Gq class Gα-protein, which stimulates phospholipase C (PLC) to yield IP₃, and subsequent Ca²⁺ release from internal stores (Zholos, A. et al., J Gen Physiol 125, 197-211 (2005); and Mauduit, P. et al., Am J Physiol 264, C1550-60 (1993)). Thus, it is likely that ANO1 activation by LPA is mediated by the PLC/IP₃ signaling pathway.

Indeed, LPA (1 µM) applied to oocytes expressing mANO1 induced a large inward current. Pretreatments with 1 µM thapsigargin (an intracellular Ca²⁺ store depleting agent) for 3 hours or 10 µM U-73122 (a PLC inhibitor; Sigma, St. Louis, MO) for 20 min before LPA application completely blocked the LPA current responses (Figs. 10A and 10B).

In addition, micro-injection of 2 mM IP₃ in 50 nl into oocytes expressing ANO1 evoked robust inward currents, whereas IP₃ injection to water-injected oocytes (control) elicited smaller though detectable currents that were comparable to those of endogenous CaCCs in oocytes (Kuruma, A. & Hartzell, H. C., Am J Physiol 276, C161-75 (1999)). Further, pretreatment of 1 µM thapsigargin blocked the IP₃-induced current in a mANO1-expressing oocyte (Figs. 10C and 10D). Thus, these results suggest that ANO1 is activated by intracellular signals that increase intracellular Ca²⁺ concentrations, most notably via the PLC/IP₃ pathway.

### Example 5: ANO1 is a chloride channel

To determine the ionic selectivity of ANO1, voltage-ramps from -80 to +80 mV were delivered to ANO1-ET_{A}R/HEK cells, in accordance with the method of Reference Example 9. Endothelin-1 (50 nM) was applied to whole cells to evoke ANO1 currents; the pipette solution contained 140 mM NMDG-Cl (0 mM Ca²⁺, 0 mM EGTA). When the bath solution (extracellular fluid) contained equimolar NMDG-Cl, a linear current-voltage relationship that reversed near zero mV (-4.5 mV) was observed (Fig. 5B). Furthermore, when the bath solution was changed to 140 mM Na-gluconate, no outward current was observed. Because NMDG⁺ and gluconate are incapable of passing through channels, these results demonstrate that ANO1 is an anion channel.

The permeability sequence of monovalent anions is a characteristic of CaCCs (Hartzell, C. et al., J., Annu Rev Physiol 67, 719-58 (2005); Kidd, J. F. & Thorn, P., Annu Rev Physiol 62, 493-513 (2000); and Frings, S., Reuter, D. & Kleene, S. J., Prog Neurobiol 60, 247-89 (2000)), and thus, the relative permeability of ANO1 to other monovalent anions was investigated. For this experiment, the pipette solution contained 140 mM NMDG-C1 (0 Ca²⁺). Permeability ratios (*P*_{X}/*P*_{Cl}) were estimated using the Goldman, Hodgkin and Katz equation from reversal potential changes induced by replacing extracellular Cl⁻ with Br⁻, I⁻, or NO₃⁻ (Arreola, J. & Melvin, J. E., J Physiol 547, 197-208 (2003)). When NaCl in the bath was substituted with equimolar NaF, NaBr, NaI, or NaN03, reversal potentials shifted from -9.0 ± 0.6 mV (n = 5) to +12.5 ± 2.9 (n = 8), -23.1 ± 2.2 (n = 5), -24.7 ± 2.5 (n = 5), and -29.1 ± 1.1 mV (n = 7), respectively (Fig. 2C). Thus, the relative permeabilities of these monovalent anions were NO₃⁻ (2.20) > I⁻ (1.85) > Br⁻ (1.74) > Cl⁻ (1.0) > F⁻(0.43), which is entirely consistent with those of endogenous CaCCs (Fig. 5B; Hartzell, C. et al., J., Annu Rev Physiol 67, 719-58 (2005), and Frings, S., Reuter, D. & Kleene, S. J., Prog Neurobiol 60, 247-89 (2000)).

LPA-induced ANO1 currents were blocked by Cl⁻ channel blockers. The Cl⁻ channel blockers were applied 10 ∼ 20 min before the LPA application. As shown in Fig. 6A, the applications of known endogenous CaCC blocking agents, 4,4'-diisothiocyanato-stilbene-2,2'-disulfonic acid (DIDS, 300 µM), niflumic acid (200 µM), or 5-nitro-2-(3-phenyl-propylamino)-benzoate (NPPB, 200 µM) (Hartzell, C. et al., J., Annu Rev Physiol 67, 719-58 (2005); Kidd, J. F. & Thorn, P., Annu Rev Physiol 62, 493-513 (2000); and Frings, S., Reuter, D. & Kleene, S. J., Prog Neurobiol 60, 247-89 (2000)) significantly blocked LPA-induced ANO1 currents in *Xenopus* oocytes (Fig. 6A). This blockade of agonist-induced ANO1 currents by DIDS, niflumic acid, or NPPB were also observed in mammalian cells transfected with mANO1. In ANO1/ET_{A}R-HEK cells, 50 nM endothelin-1 was applied twice with a 5 min interval, and second responses were found to reach 87.1 ± 11.4% (n = 12) of first responses, eliciting weak tachyphylaxis. However, pretreatment or co-application of DIDS (10 µM), miflumic acid (10 µM), and NPPB (10 µM) markedly inhibited this response of ANO1/ET_{A}R-HEK cells to the second application of endothelin-1 (Fig. 6B).

### Example 6: Inhibition by CaCC-specific inhibitors

In addition, modifiers of various enzymes or transporters such as fluoxetine (serotonin reuptake inhibitor), tamoxifen (estrogen receptor modulator), n-phenyl-anthranilic acid (NPA, cyclooxygenase inhibitor), and mefloquine (antimalarial drug) are known to block native CaCCs (Eggermont, J., Proc Am Thorac Soc. 1, 22-7 (2004); Nilius, B. et al. J Physiol. 498, 381-96 (1997); Nilius, B. & Droogmans, G., Acta Physiol Scand 177, 119-47 (2003)). If ANO1 is a CaCC, mANO1 should be inhibited by these CaCC blockers. Indeed, these chemicals markedly inhibited endothelin-1 induced currents in ANO1/ET_{A}R-HEK cells when tested in accordance with the method of Example 5 (Fig. 6B).

### Example 7: Activation of ANO1 by intracellular Ca²⁺

To prove that ANO1 is activated by Ca²⁺, Ca²⁺ was applied to inside-out membrane patches isolated from ANO1/ET_{A}R-HEK cells. Ca²⁺ in various concentrations applied to the bath (intracellular side) evoked macroscopic single-channel currents in a dose-dependent manner (E_{hold} = -60 mV) (Fig. 7A). A half-maximal concentration (EC₅₀) for ANO1 activation was 2.6 µM at -60 mV. The threshold concentration of Ca²⁺ reached about 0.3 µM at -60 mV holding potential (Fig. 7B). Remarkably, ANO1 activation by Ca²⁺ was voltage-dependent, which is also characteristic of endogenous CaCCs (Nilius, B. et al., Cell Calcium 22, 53-63 (1997), Kuruma, A. & Hartzell, H. C., J Gen Physiol 115, 59-80 (2000), Evans, M. G. & Marty, A., J Physiol 378, 437-60 (1986)). At a depolarized membrane potential (+60 mV), ANO1 was more sensitive to Ca²⁺ and the concentration-response curve shifted to the left, resulting in an EC₅₀ of 0.4 µM (Fig. 7B). Hill's coefficients of the concentration-response curves were 2.0 and 2.4 at -60 and +60 mV, respectively.

The amplitudes of single-channel ANO1 currents were small, like those of endogenous CaCCs (Nilius, B. et al., J Physiol 498, 381-96 (1997), Piper, A. S. & Large, W. A., J Physiol 547, 181-96 (2003)). At -60 mV, the average single-channel current amplitude was 0.42 ± 0.01 pA (n = 6) (Fig. 7C). Moreover, the single-channel current amplitudes of ANO1 activated by Ca²⁺ was measured at various membrane potentials, and a linear current-voltage relationship was found. In addition, the slope conductance of ANO1 was 8.3 pS, which is comparable to those of native CaCCs in various cell types (Nilius, B. et al., *supra,* Piper, A. S. & Large, W. A., *supra*)*.*

### Example 8: Expression pattern

To visualize the tissue distribution of ANO1, an ANO1 polyclonal antibody was generated for immunohistochemical analysis, in accordance with the method of Reference Example 6. Residues 451 to 466 were chosen for this purpose, which are located in the intracellular loop between transmembrane domains 2 and 3 (Fig. 2B). Further, immunohistochemical staining of various tissues using the polyclonal antibody was carried out in accordance with the method of Reference Example 8. As expected for a CaCC candidate, ANO1 was found to be expressed in tissues with CaCC activity.

As shown in Fig. 8A, dense ANO1 immunoreactivity was observed in epithelial cells of pulmonary bronchioles, whereas expression in lung alveolar cells was less prominent. Pancreatic acinar cells were also heavily stained by ANO1 antibody (Fig. 8B). In the kidney, dense immunoreactivity was observed mainly in the epithelia of proximal (black arrow) and weakly in distal renal tubules (arrowhead) (Fig. 8C). Remarkably, as has been proven by electrophysiological experiments (Maricq, A. V. & Korenbrot, J. I., Neuron 1, 503-15 (1988), dense ANO1 immunoreactivity was found in all retinal cell layers (Fig. 8D), i.e., the outer nuclear layer, which contains the bodies of photoreceptors (arrow head), the inner nuclear and ganglion cell layers (arrow). Most sensory neurons but not satellite cells in dorsal root ganglia stained positively for ANO1 (Fig. 8E) which is in agreement with the modulatory effects of CaCCs in sensory transmission (Kenyon, J. L. & Scott, R. H., Calcium-Activated Chloride Channels (ed. Fuller, C. M.) 135-166 (Academic Press, Amsterdam, 2002)). Small sensory neurons (arrow) tended to stain more densely than large sensory neurons (arrowhead), suggesting that ANO1 has a modulatory role in nociception, because small neurons in dorsal-root ganglia are intimately involved in nociception (Willis, W. D. & Coggeshall, R. E., Sensory Mechanisms of the Spinal Cord (ed.) (Plenum Press, New York, 2004)). In submandibular glands, dense immunoreactivity was observed at the apices of acinar cells (Fig. 8F).

ANO1 was also expressed in skin (Fig. 8G), especially in keratinocytes (arrowhead) and in the sebaceous glands (black arrow). Moderate ANO1 expression was also present in cardiac ventricular myocytes (Fig. 8H).

In contrast, ANO1 immunoreactivity was not found in specific nuclei in the brain, instead, weak immunoreactivity was found in neural tracts. Furthermore, dense immunoreactivity was observed in Leydig cells and moderate immunoreactivity was observed in growing spermatocytes in testes. Thus, the expression pattern of ANO1 in these secretory epithelial cells, cardiac ventricular myocytes, and in retinal and sensory neurons was found to coincide with the reported locations of CaCCs (Hartzell, C. et al., J., Annu Rev Physiol 67, 719-58 (2005); and Frings, S., Reuter, D. & Kleene, S. J., Prog Neurobiol 60, 247-89 (2000)).

### Example 9: Current recording in hANO1

To determine whether hANO1 is activated by GPCR stimulation, hANO1 cloned in accordance with the method of Reference Example 1 was expressed in HEK cells along with ET_{A}R. As shown in Fig. 11, application of endothelin-1 (50 nM) to hANO1/ET_{A}R-HEK cells evoked large Cl⁻ currents. Furthermore, hANO1 responded to repeated stimulations by endothelin-1, eliciting a small tachyphylaxis. Thus, like mANO1, hANO1 is also activated by receptor stimulation.

### Example 10: Current recording in mouse ANO1 stable cell line

To detect active compounds from chemical libraries or other sources of chemicals, a stable cell line, that constitutively expresses mouse ANO1, was constructed in accordance with the method of Reference Example 7. To construct a stable cell line, mouse ANO1 DNA was incorporated to the genomic DNA of human embryonic kidney (HEK) cells.

As shown in Fig. 12, application of 500 nM ionomycin (a Ca²⁺ ionophore) caused robust inward current in the stable cell line. This confirms that ANO1 is constitutively expressed in the stable cell line.

### Example 11: Monitoring activity of ANO1 by voltage-sensitive fluorescent dye in ANO1

To determine if ANO1 is applicable for high-throughput screening (HTS) analysis, we constructed a stable cell line that constitutively expresses mANO1 (Reference example 7). The mANO1 stable cell line was grown in wells of a 96-well plate. mANO1-stably expressing HEK cells were incubated with a voltage sensor fluorescent dye (FLIPR^{®}-membrane potential assay kit, Molecular Device) to detect change in membrane potential when ANO1 is activated. When ANO1 is activated, Cl⁻ would flux out the cell to depolarize the membrane, which makes the fluorescence dye fluoresce. After wash out the fluorescent dye, 100 µM ATP was applied. As shown in Fig. 13A, the application of ATP to mANO1-stably expressing HEK cells (left panel) evoked robust change in membrane potentials detected by fluorescent intensity, but not in control HEK cells (right panel). Fluorescent intensity was detected at excitation 530 nm and emission 565 nm by FlexStation 2 (Molecular device). In addition, as shown in Fig. 13B, application of a Ca²⁺ ionophore, A23187, that increases intracellular Ca²⁺, evoked membrane potential change in ANO1-stably expressing HEK cells (left panel), but not in control HEK cells (right panel). F/F₀: ratio of fluorescence intensity. Pretreatment of 2APB, a known blocker of IP₃ receptor, blocked the ATP induced-membrane potential change in ANO1-stably expressing HEK cells (Fig. 13C). These results now indicate that the protocol using the fluorescent dye and the stable cell line is suitable for high throughput screening analysis and also applicable to screen out chemicals that modify activity of ANO1.

## Claims

1. An in vitro method of identifying an agent which modulates an activity of the protein TMEM16A, wherein the protein is represented by the amino acid sequence of SEQ ID NO: 1 and transports chloride ions across a cell membrane, comprising:
(a) exposing cells that express the protein to the agent; and
(b) measuring degree of chloride ion transport in the exposed cells, wherein a change in the degree of chloride ion transport compared to control cells, which express the protein but not exposed to the agent, is indicative of an agent capable of modulating an activity of the protein.

2. The method of claim 1, wherein the cells expressing the protein are prepared by transfecting cells with a nucleic acid encoding the protein.

3. The method of claim 1, wherein the agent increases or reduces the degree of chloride ion transport.

4. An in vitro method of identifying an agent which inhibits an activity of the protein TMEM16A, wherein the protein is represented by the amino acid sequence of SEQ ID NO: 1 and transports chloride ions across a cell membrane, comprising:
(a) exposing cells that express the protein and a G-protein coupled receptor (GPCR) to the agent;
(b) applying a ligand of the GPCR to the resulting cells; and
(c) measuring degree of chloride ion transport in the exposed cells, wherein a reduction in the degree of chloride ion transport compared to control cells, which express the protein and GPCR but not exposed to the agent, is indicative of an agent capable of inhibiting the activity of the protein.

5. The method of claim 4, wherein the cells expressing the protein and the GPCR are prepared by transfecting cells with nucleic acids encoding the protein and the GPCR, respectively.

6. The method of claim 2 or 5, wherein the nucleic acid consists of the nucleotide sequence of SEQ ID NO: 2 or 4.

7. The method of claim 1 or 4, wherein the cells are eukaryotic cells.

8. The method of claim 7, wherein the cells are mammalian cells.

9. The method of claim 8, wherein the cells are selected from the group consisting of human embryonic kidney (HEK) cells, HEK 293 cells (ATCC CRL 1573), 3T3-L1 cells (ATCC CL 173), C6 cells (ATCC CCL 107), Chinese hamster ovary (CHO) cells (ATCC CCL61), CHO-K1 cells (ATCC CCL 61), NIH Swiss mouse embryo cells NIH/3T3 (ATCC CRL 1658), baby hamster kidney cells (BHK), COS-1 cells (ATCC CRL 1650), COS-7 cells (ATCC CRL 1651), HaCaT cells, HeLa cells (ATCC CCL 2), HeLa S3 cells (ATCC CCL 2.2), HepG2 cells (ATCC HB 8065), HL-60 cells (ATCC CCL 240), HUV-EC-C cells (ATCC CRL 1730), Jurkat cells (ATCC TIB 152), K-562 cells (ATCC CCL 243), L6 cells (ATCC CRL 1458), MCF7 cells (ATCC HTP 22), MDCK cells (ATCC CCL 34), NIH/3T3 cells (ATCC CRL 1658), RAW 264.7 cells (ATCC TIB 71), RBL-1 cells (ATCC CRL 1378), SH-SY5Y cells (ATCC CRL 2266), U-937 cells (ATCC CRL 1593.2) and the like eukaryotic tissue culture cell lines.

10. The method of claim 1 or 4, wherein the agent is selected from the group consisting of chemical compounds, natural products, oligonucleotides, peptides and antibodies.

## Patentansprüche

1. In-vitro Verfahren zur Identifizierung eines Mittels, welches eine Aktivität des Proteins TMEM16A moduliert, wobei das Protein durch die Aminosäuresequenz von SEQ ID NO: 1 dargestellt ist und Chloridionen über eine Zellmembran transportiert, wobei das Verfahren umfasst:
(a) Inkontaktbringen von Zellen, welche das Protein exprimieren, mit dem Mittel; und
(b) Messen des Grades des Chloridionen-Transports in den exponierten Zellen, wobei eine Änderung in dem Grad des Chloridionen-Transports im Vergleich zu Kontrollzellen, welche das Protein zwar exprimieren, dem Mittel aber nicht ausgesetzt sind, indikativ ist für ein Mittel, welches in der Lage ist, eine Aktivität des Proteins zu modulieren.

2. Verfahren nach Anspruch 1, wobei die Zellen, welche das Protein exprimieren, durch Transfektion der Zellen mit einer Nukleinsäure, welche das Protein kodiert, hergestellt werden.

3. Verfahren nach Anspruch 1, wobei das Mittel den Grad des Chloridionen-Transports erhöht oder verringert.

4. In-vitro Verfahren zur Identifizierung eines Mittels, welches eine Aktivität des Proteins TMEM16A inhibiert, wobei das Protein durch die Aminosäuresequenz von SEQ ID NO: 1 dargestellt ist und Chloridionen über eine Zellmembran transportiert, wobei das Verfahren umfasst:
(a) Inkontaktbringen von Zellen, welche das Protein und einen G-Protein-gekoppelten Rezeptor (GPCR) exprimieren, mit dem Mittel;
(b) Applizieren eines Liganden des GPCR zu den resultierenden Zellen; und
(c) Messen des Grades des Chloridionen-Transports in den exponierten Zellen, wobei eine Verringerung im des Grades des Chloridionen-Transports im Vergleich zu Kontrollzellen, welche das Protein und den GPCR zwar exprimieren, dem Mittel aber nicht ausgesetzt sind, indikativ für ein Mittel ist, welches in der Lage ist, die Aktivität des Proteins zu inhibieren.

5. Verfahren nach Anspruch 4, wobei die Zellen, welche das Protein und den GPCR exprimieren, durch Transfektion von Zellen mit Nukleinsäuren, welche das Protein bzw. den GPCR kodieren, hergestellt werden.

6. Verfahren nach Anspruch 2 oder 5, wobei die Nukleinsäure aus der Nukleotidsequenz von SEQ ID NO: 2 oder 4 besteht.

7. Verfahren nach Anspruch 1 oder 4, wobei die Zellen eukaryontische Zellen sind.

8. Verfahren nach Anspruch 7, wobei die Zellen Säugetierzellen sind.

9. Verfahren nach Anspruch 8, wobei die Zellen ausgewählt sind aus der Gruppe bestehend aus menschlichen embryonalen Nieren (HEK)- Zellen, HEK 293 Zellen (ATCC CRL 1573), 3T3-L1 Zellen (ATCC CL 173), C6 Zellen (ATCC CCL 107), chinesischen Hamsterovar (CHO)-Zellen (ATCC CCL61), CHO-K1 Zellen (ATCC CCL 61), NIH Schweizer Mausembryozellen NIH/3T3 (ATCC CRL 1658), Babyhamster-Nierenzellen (BHK), COS-1 Zellen (ATCC CRL 1650), COS-7 Zellen (ATCC CRL 1651), HaCaT-Zellen, HeLa-Zellen (ATCC CCL 2), HeLa S3-Zellen (ATCC CCL 2.2), HepG2-Zellen (ATCC HB 8065), HL-60-Zellen (ATCC CCL 240), HUV-EC-C-Zellen (ATCC CRL 1730), Jurkat-Zellen (ATCC TIB 152), K-562-Zellen (ATCC CCL 243), L6-Zellen (ATCC CRL 1458), MCF7-Zellen (ATCC HTP 22), MDCK-Zellen (ATCC CCL 34), NIH/3T3-Zellen (ATCC CRL 1658), RAW 264.7-Zellen (ATCC TIB 71), RBL-1-Zellen (ATCC CRL 1378), SH-SY5Y-Zellen (ATCC CRL 2266), U-937-Zellen (ATCC CRL 1593.2) und ähnliche eukaryontische Gewebekulturzelllinien.

10. Verfahren nach Anspruch 1 oder 4, wobei das Mittel ausgewählt ist aus der Gruppe bestehend aus chemischen Verbindungen, natürlichen Produkten, Oligonukleotiden, Peptiden und Antikörpern.

## Revendications

1. Procédé *in vitro* d'identification d'un agent qui module une activité de la protéine TMEM16A, la protéine étant représentée par la séquence d'acides aminés de SEQ ID n° : 1 et qui transporte les ions chlore à travers une membrane cellulaire, comprenant :
(a) l'exposition des cellules qui expriment la protéine à l'agent ; et
(b) la mesure du degré de transport des ions chlore dans les cellules exposées, un changement dans le degré de transport des ions chlore comparé aux cellules témoins, qui expriment la protéine mais ne sont pas exposées à l'agent, étant indicateur d'un agent capable de moduler une activité de la protéine.

2. Procédé selon la revendication 1, dans lequel les cellules exprimant la protéine sont préparées en transfectant les cellules avec un acide nucléique codant pour la protéine.

3. Procédé selon la revendication 1, dans lequel l'agent augmente ou réduit le degré de transport des ions chlore.

4. Procédé *in vitro* d'identification d'un agent qui inhibe une activité de la protéine TMEM16A, la protéine étant représentée par la séquence d'acides aminés de SEQ ID n° : 1 et qui transporte les ions chlore à travers une membrane cellulaire, comprenant :
(a) l'exposition des cellules qui expriment la protéine et d'un récepteur couplé à la protéine G (GPCR) à l'agent ;
(b) l'application d'un ligand du GPCR aux cellules résultantes ; et
(c) la mesure du degré de transport des ions chlore dans les cellules exposées, une réduction du degré de transport des ions chlore comparé aux cellules témoins, qui expriment la protéine et le GPCR mais ne sont pas exposées à l'agent, étant indicative d'un agent capable d'inhiber l'activité de la protéine.

5. Procédé selon la revendication 4, dans lequel les cellules exprimant la protéine et le GPCR sont préparées par transfection des cellules avec des acides nucléiques codant respectivement pour la protéine et le GPCR.

6. Procédé selon la revendication 2 ou 5, l'acide nucléique étant constitué de la séquence de nucléotides de SEQ ID n° : 2 ou 4.

7. Procédé selon la revendication 1 ou 4, dans lequel les cellules sont des cellules eucaryotes.

8. Procédé selon la revendication 7, dans lequel les cellules sont des cellules de mammifère.

9. Procédé selon la revendication 8, dans lequel les cellules sont sélectionnées dans le groupe constitué des cellules de rein embryonnaire humain (HEK), des cellules HÉK 293 (ATCC CRL 1573), des cellules 3T3-L1 (ATCC CL 173), des cellules C6 (ATCC CCL 107), des cellules d'ovaire de hamster chinois (CHO) (ATCC CCL61), des cellules CHO-K1 (ATCC CCL 61), des cellules d'embryon de souris suisse NIH NIH/3T3 (ATCC CRL 1658), des cellules de rein de hamster nouveau-né (BHK), des cellules COS-1 (ATCC CRL 1650), des cellules COS-7 (ATCC CRL 1651), des cellules HaCaT, des cellules HeLa (ATCC CCL 2), des cellules HeLa S3 (ATCC CCL 2.2), des cellules HepG2 (ATCC HB 8065), des cellules HL-60 (ATCC CCL 240), des cellules HUV-EC-C (ATCC CRL 1730), des cellules Jurkat (ATCC TIB 152), des cellules K-562 (ATCC CCL 243), des cellules L6 (ATCC CRL 1458), des cellules MCF7 (ATCC HTP 22), des cellules MDCK (ATCC CCL 34), des cellules NIH/3T3 (ATCC CRL 1658), des cellules RAW 264.7 (ATCC TIB 71), des cellules RBL-1 (ATCC CRL 1378), des cellules SH-SY5Y (ATCC CRL 2266), des cellules U-937 (ATCC CRL 1593.2) et des lignées cellulaires semblables de culture de tissu eucaryote.

10. Procédé selon la revendication 1 ou 4, dans lequel l'agent est sélectionné dans le groupe constitué des composés chimiques, des produits d'origine naturelle, des oligonucléotides, des peptides et des anticorps.
